(19) Europäisches Patentamt
European Patent Office
Office européen des brevets

(11) **EP 2 848 183 A1**

(12) **EUROPEAN PATENT APPLICATION**
published in accordance with Art. 153(4) EPC

(43) Date of publication:
**18.03.2015   Bulletin 2015/12**

(51) Int Cl.:
***A61B 1/00*** *(2006.01)*       ***A61B 5/07*** *(2006.01)*

(21) Application number: **13786895.6**

(22) Date of filing: **07.05.2013**

(86) International application number:
**PCT/JP2013/062851**

(87) International publication number:
**WO 2013/168710 (14.11.2013 Gazette 2013/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority:   **07.05.2012   JP 2012106330**

(71) Applicant: **Olympus Medical Systems Corp.
Tokyo 151-0072 (JP)**

(72) Inventors:
 • **CHIBA, Atsushi
   Tokyo 151-0072 (JP)**
 • **TAKIZAWA, Hironobu
   Tokyo 151-0072 (JP)**
 • **KAWANO, Hironao
   Tokyo 151-0072 (JP)**

(74) Representative: **Schicker, Silvia
   Wuesthoff & Wuesthoff
   Patent- und Rechtsanwälte
   Schweigerstrasse 2
   81541 München (DE)**

(54) **GUIDE DEVICE**

(57)     There is provided a guidance device including a permanent magnet having a shape that allows generation of a magnetic field suitable for guiding a capsule medical apparatus. The guidance device applies a magnetic field to a capsule endoscope 10 within which a first permanent magnet is arranged when the capsule endoscope 10 is introduced into a subject, to guide the capsule endoscope 10 within the subject. The guidance device includes an extracorporeal permanent magnet 25a configured to be disposed outside the subject. The extracorporeal permanent magnet 25a is a second permanent magnet that has a first plane containing a magnetization direction and a first direction orthogonal to the magnetization direction and confines the capsule endoscope 10 within a region facing the first plane. In the extracorporeal permanent magnet 25a, the length in the first direction is longer than the length in the magnetization direction.

FIG.4

**Description**

Field

The present invention relates to a guidance device for guiding a capsule medical apparatus introduced into a subject.

Background

In the field of an endoscope, there has been promoted the development of a capsule endoscope having a size that allows for being introduced into the gastrointestinal tract of a subject such as a patient. The capsule endoscope has an imaging function and a wireless communication function inside a capsule-shaped casing. The capsule endoscope is swallowed through the mouth of a subject, and thereafter travels in the gastrointestinal tract by peristaltic movement or the like. Meanwhile, image data of the inside of the organ of a subject (hereinafter, also referred to as an in-vivo image) is sequentially acquired, and wirelessly transmitted to a receiving device placed outside a subject. The image data received by a receiving device is incorporated in an image display apparatus to be subjected to certain image processing. Accordingly, an in-vivo image is displayed as a still image or a moving image on a display screen. A user such as a medical doctor or a nurse observes the in-vivo image displayed on the image display apparatus in this manner, and diagnoses the condition of the organ of a subject.

There is recently proposed a guidance system that guides with a magnetic force (hereinafter, referred to as magnetically guides) a capsule endoscope introduced into a subject (for example, see Patent Literature 1 and Patent Literature 2). Generally, in such a guidance system, a permanent magnet is disposed inside the capsule endoscope, and a guidance device including a magnetic field generation unit such as an electromagnet is disposed outside a subject. A magnetic field generated by the magnetic field generation unit is applied to the permanent magnet provided inside the capsule endoscope. With a magnetic attracting force caused by the generated magnetic field, the capsule endoscope is magnetically guided to a desired position.

Also, there is another guidance device provided with a display unit that receives image data acquired by the capsule endoscope and displays an in-vivo image; an input device that operates the position or posture of the capsule endoscope; and the like. In the case of such a guidance device, a user can operate the magnetic guidance of the capsule endoscope using the input device while referring to the in-vivo image displayed on the display unit.

Also, there has been developed a system that applies a magnetic field from the outside of a subject to the capsule endoscope to perform signal control such as on/off of a switch for the capsule endoscope (for example, see Patent Literature 3).

Citation List

Patent Literature

Patent Literature 1: Japanese Patent Application Laid-open No. 2006-68501
Patent Literature 2: JP-T-2008-503310
Patent Literature 3: WO 2007/083708

Summary

Technical Problem

Here, when a permanent magnet is used as a magnetic field generation unit that generates a magnetic field for guiding the capsule medical apparatus from the outside of a subject, the permanent magnet, unlike an electromagnet, cannot adjust the strength and distribution of the magnetic field to be generated. For this reason, in order to efficiently guide the capsule medical apparatus, the shape and the like of the permanent magnet capable of generating a magnetic field suitable for the guidance need to be previously defined.

The present invention has been made in view of the foregoing and an object of the invention is to provide a guidance device including a permanent magnet having a shape that allows generation of a magnetic field suitable for guiding a capsule medical apparatus.

Solution to Problem

To solve the above-mentioned problem and achieve the object, a guidance device according to the invention is a guidance device for applying a magnetic field to a capsule medical apparatus within which a first permanent magnet is arranged when the capsule medical apparatus is introduced into a subject, to guide the capsule medical apparatus within the subject. The guidance device includes a second permanent magnet configured to be disposed outside the subject, the second permanent magnet having a first plane containing a magnetization direction and a first direction orthogonal to the magnetization direction, and being configured to confine the capsule medical apparatus within a region facing the first plane. The second permanent magnet has a length in the first direction longer than a length in the magnetization direction.

In the above-described guidance device, the length in the magnetization direction is 2/3 or less of the length in the first direction.

In the above-described guidance device, a length in a second direction orthogonal to the magnetization direction and the first direction in the second permanent magnet is shorter than the length in the first direction.

In the above-described guidance device, a value K given by

$$K = \sqrt{\frac{L_y^2}{L_x \times L_z}}$$

is more than 1 and 22.6 or less, where $L_x$ is the length in the magnetization direction, $L_y$ is the length in the first direction, and $L_z$ is a length in a second direction orthogonal to the magnetization direction and the first direction.

In the above-described guidance device, the length in the magnetization direction is the length in the second direction or longer.

The above-described guidance device further includes a revolution angle changing unit configured to rotate the second permanent magnet around an axis orthogonal to an optional reference plane parallel to the first direction.

The above-described guidance device further includes a translation mechanism configured to translate the second permanent magnet within an optional reference plane parallel to the first direction.

The above-described guidance device further includes an elevation angle changing unit configured to rotate the second permanent magnet around an axis parallel to the first direction, to change an elevation angle of the magnetization direction with respect to an optional reference plane parallel to the first direction.

In the above-described guidance device, the reference plane is parallel to a horizontal plane.

The above-described guidance device further includes a shielding unit configured to shield a magnetic field generated by the second permanent magnet in an effective magnetic field area as an area where the magnetic field capable of guiding the capsule medical apparatus is generated by the second permanent magnet, and configured to switch between a first state in which the magnetic field is not shielded in the effective magnetic field area and a second state in which the magnetic field is shielded in the effective magnetic field area.

In the above-described guidance device, the shielding unit includes: a magnetic body; and a drive unit configured to insert and remove the magnetic body into and from between the second permanent magnet and the effective magnetic field area.

The above-described guidance device further includes a placing table on which the subject is configured to be placed, the placing table having a first region on which an examination target region of the subject is configured to be placed and a second region on which a non-examination target region of the subject is configured to be placed. The magnetic body is arranged within the second region on the placing table, and the drive unit is configured to drive the magnetic body via the placing table.

In the above-described guidance device, the shielding unit includes: a magnetic fluid; a magnetic fluid housing unit that is disposed between the second permanent magnet and the effective magnetic field area and is configured to house the magnetic fluid; a magnetic fluid storage unit that communicates with the magnetic fluid housing unit; and a magnetic fluid moving unit configured to move the magnetic fluid between the magnetic fluid housing unit and the magnetic fluid storage unit.

The above-described guidance device further includes: a placing table on which the subject is configured to be placed, the placing table having a first region on which an examination target region of the subject is configured to be placed and a second region on which a non-examination target region of the subject is configured to be placed; and a drive unit configured to drive the placing table to switch a position of the placing table between a first position where the first region is inserted between the second permanent magnet and the effective magnetic field area and a second position where the first region is removed from between the second permanent magnet and the effective magnetic field area. The shielding unit includes: a magnetic fluid; a magnetic fluid housing unit that is disposed in a region which is inserted between the second permanent magnet and the effective magnetic field area when the placing table is in the second position, and is configured to house the magnetic fluid; a magnetic fluid storage unit that communicates with the magnetic fluid housing unit; and a magnetic fluid moving unit configured to work in conjunction with the drive unit and configured to move the magnetic fluid between the magnetic fluid housing unit and the magnetic fluid storage unit. The magnetic fluid moving unit moves the magnetic fluid to the magnetic fluid housing unit when the placing table is shifted from the first position to the second position.

In the above-described guidance device, the magnetic fluid moving unit is a piston that is disposed in the magnetic fluid storage unit for changing a volume within the magnetic fluid storage unit to inject the magnetic fluid from the magnetic fluid storage unit into the magnetic fluid housing unit or for sucking the magnetic fluid from the magnetic fluid housing unit into the magnetic fluid storage unit.

The above-described guidance device further includes: a detecting unit configured to detect a shielding state of the magnetic field by the shielding unit; and a control unit configured to control switching between the first state and the second state depending on a detection result by the detecting unit and a status of an examination by the capsule medical apparatus.

The above-described guidance device further includes: a detecting unit configured to detect a shielding state of the magnetic field by the shielding unit; and a notifying unit configured to notify a detection result by the detecting unit.

Advantageous Effects of Invention

According to the invention, the length the second permanent magnet in the first direction is longer than the length of the second permanent magnet in the magnetization direction. Therefore, the magnetic field suitable for guiding the capsule medical apparatus can be generated by the second permanent magnet. As a result, it is possible to achieve a guidance device that can efficiently guide a capsule medical apparatus.

Brief Description of Drawings

FIG. 1 is a diagram illustrating a configuration example of a capsule medical apparatus guidance system according to a first embodiment of the present invention.

FIG. 2 is a partial sectional side view schematically illustrating a configuration example when the guidance device illustrated in FIG. 1 is in a magnetic field generating state.

FIG. 3 is a partial sectional side view schematically illustrating a configuration example when the guidance device illustrated in FIG. 1 is in a magnetic field shielding state.

FIG. 4 is a schematic diagram for explaining an installed state of the extracorporeal permanent magnet illustrated in FIG. 2.

FIG. 5 is a cross-sectional schematic diagram illustrating an example of an internal configuration of the capsule endoscope illustrated in FIG. 1.

FIG. 6 is a schematic diagram for explaining a relative positional relationship between an imaging element and a permanent magnet inside a capsule endoscope.

FIG. 7 is a conceptual diagram for explaining a situation of a capsule endoscope when a liquid is introduced into a subject (a state in which a magnetic field is not allowed to act).

FIG. 8 is a conceptual diagram for explaining a situation of a capsule endoscope when a liquid is introduced into a subject (a state in which a magnetic field is allowed to act).

FIG. 9 is a diagram illustrating an example of an image displayed on a display screen of the display unit illustrated in FIG. 1.

FIG. 10 is a schematic diagram for explaining a position control method in a horizontal direction of a capsule endoscope.

FIG. 11 is a schematic diagram for explaining a position control method in a vertical direction of a capsule endoscope.

FIG. 12 is a diagram illustrating an example of the operation input unit illustrated in FIG. 1.

FIG. 13 is a diagram for explaining magnetic guidance of a capsule endoscope operable by the operation input unit illustrated in FIG. 1.

FIG. 14 is a diagram exemplifying a menu screen displayed on a display unit.

FIG. 15 is a flow chart illustrating action of the capsule medical apparatus guidance system illustrated in FIG. 1.

FIG. 16 is a partial sectional side view schematically illustrating a configuration example when the guidance device illustrated in FIG. 1 is in a weak magnetic field generating state.

FIG. 17 is a schematic diagram for explaining evaluation items in a simulation of calculating the relationship between the shape of an extracorporeal permanent magnet and the generated magnetic field.

FIG. 18 is a table illustrating a ratio in length among sides of a permanent magnet used in the simulation.

FIG. 19 is a graph illustrating magnetic field strengths of the permanent magnets illustrated in FIG. 18.

FIG. 20 is a graph illustrating magnetic field gradients in the z-axis direction generated by the permanent magnets illustrated in FIG. 18.

FIG. 21 is a graph illustrating magnetic field gradients in the x-axis direction generated by the permanent magnets illustrated in FIG. 18.

FIG. 22 is a graph illustrating magnetic field gradients in the y-axis direction generated by the permanent magnets illustrated in FIG. 18.

FIG. 23 is a table illustrating a ratio in length among sides of each permanent magnet used in another simulation.

FIG. 24 is a graph illustrating magnetic field strengths of the permanent magnets illustrated in FIG. 23.

FIG. 25 is a graph illustrating magnetic field gradients in the z-axis direction generated by the permanent magnets illustrated in FIG. 23.

FIG. 26 is a graph illustrating magnetic field gradients in the x-axis direction generated by the permanent magnets illustrated in FIG. 23.

FIG. 27 is a graph illustrating magnetic field gradients in the y-axis direction generated by the permanent magnets illustrated in FIG. 23.

FIG. 28 is a graph illustrating a relationship between the ratio of a length in the y-axis direction to a length in the z-axis direction, and the ratio of a magnetic field strength of a permanent magnet having each dimensional ratio to a magnetic field strength of a permanent magnet of type y-x-z (33).

FIG. 29 is a table illustrating a ratio in length among sides of each permanent magnet used in further simulation.

FIG. 30 is a graph illustrating a relationship between a length in the z-axis direction and a magnetic field strength of each permanent magnet illustrated in FIG. 29.

FIG. 31 is a graph illustrating a relationship between a length in the z-axis direction and a magnetic field gradient in the z-axis direction of each permanent magnet illustrated in FIG. 29.

FIG. 32 is a graph illustrating a relationship between a length in the z-axis direction and a magnetic field gradient in the x-axis direction of each permanent magnet illustrated in FIG. 29.

FIG. 33 is a graph illustrating a relationship between a length in the z-axis direction and a magnetic field gradient in the y-axis direction of each permanent magnet illustrated in FIG. 29.

FIG. 34 is a table illustrating evaluation of the results indicated in FIG. 30 to FIG. 33.

FIG. 35 is a diagram illustrating an example of the operation input unit according to modified example 1-1.

FIG. 36 is a diagram for explaining magnetic guidance of a capsule endoscope operable by the operation input unit illustrated in FIG. 35.

FIG. 37 is a schematic diagram illustrating a modified example of the magnetic field generating unit illustrated in FIG. 1.

FIG. 38 is a partial sectional side view schematically illustrating a configuration example (in a magnetic field generating state) of the capsule medical apparatus guidance system according to a second embodiment of the present invention.

FIG. 39 is a partial sectional side view schematically illustrating a configuration example (in a magnetic field shielding state) of the capsule medical apparatus guidance system according to the second embodiment of the present invention.

FIG. 40 is a partial sectional side view schematically illustrating a configuration example (in a magnetic field generating state) of the capsule medical apparatus guidance system according to a third embodiment of the present invention.

FIG. 41 is a partial sectional side view schematically illustrating a configuration example (in a magnetic field shielding state) of the capsule medical apparatus guidance system according to the third embodiment of the present invention.

FIG. 42 is a partial sectional side view schematically illustrating a configuration example (in a magnetic field generating state) of the capsule medical apparatus guidance system according to a fourth embodiment of the present invention.

FIG. 43 is a partial sectional side view schematically illustrating a configuration example (in a magnetic field shielding state) of the capsule medical apparatus guidance system according to the fourth embodiment of the present invention.

FIG. 44 is a perspective view schematically illustrating a configuration example of the capsule medical apparatus guidance system according to a fifth embodiment of the present invention.

Description of Embodiments

A guidance device according to embodiments of the present invention will be described below with reference to the drawings. Although the following description illustrates a guidance system for a capsule endoscope that uses, as a capsule medical apparatus, a capsule endoscope that is orally introduced into a subject and floats in a liquid stored in the stomach of a subject, the embodiment does not limit the present invention. That is, in the present invention, there can be used various capsule medical apparatuses such as a capsule endoscope that moves in the lumen from the esophagus to the anus of a subject, and a capsule endoscope to be inserted together with an isotonic solution from the anus. Also, in the following description, the shape, the size and the positional relationship in each drawing are merely schematically illustrated to a degree that facilitates understanding of a content of the present invention. Therefore, the present invention is not limited only by the shape, the size and the positional relationship illustrated in each drawing. Here, in the description of the drawings, the same signs are assigned to the same parts.

(First Embodiment)

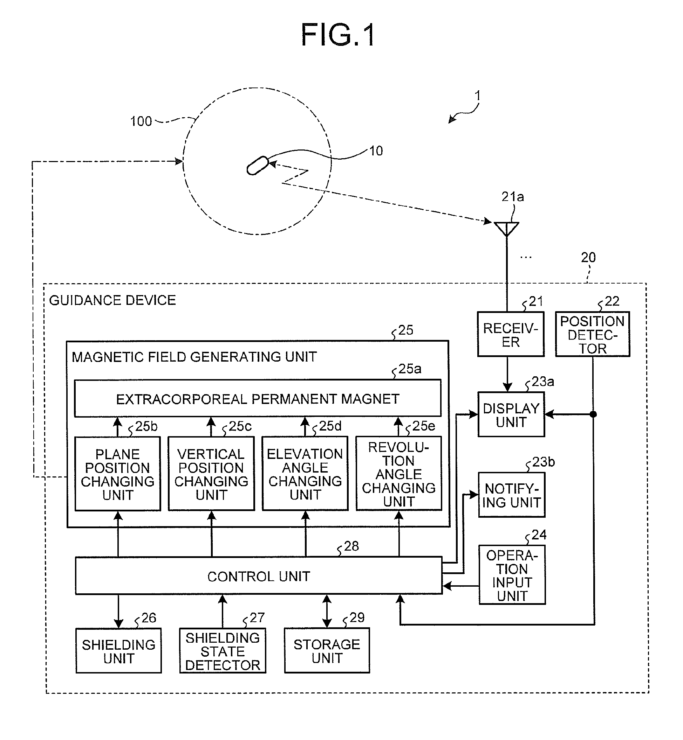

FIG. 1 is a schematic diagram illustrating a configuration example of a capsule medical apparatus guidance system according to a first embodiment of the present invention. As illustrated in FIG. 1, a capsule medical apparatus guidance system (hereinafter, also merely referred to as a guidance system) 1 according to the first embodiment includes a capsule endoscope 10 and a guidance device 20. The capsule endoscope 10 is a capsule medical apparatus to be introduced into the body cavity of a subject, and contains a permanent magnet therein. The guidance device 20 generates a three-dimensional magnetic field to magnetically guide the capsule endoscope 10 introduced into the subject.

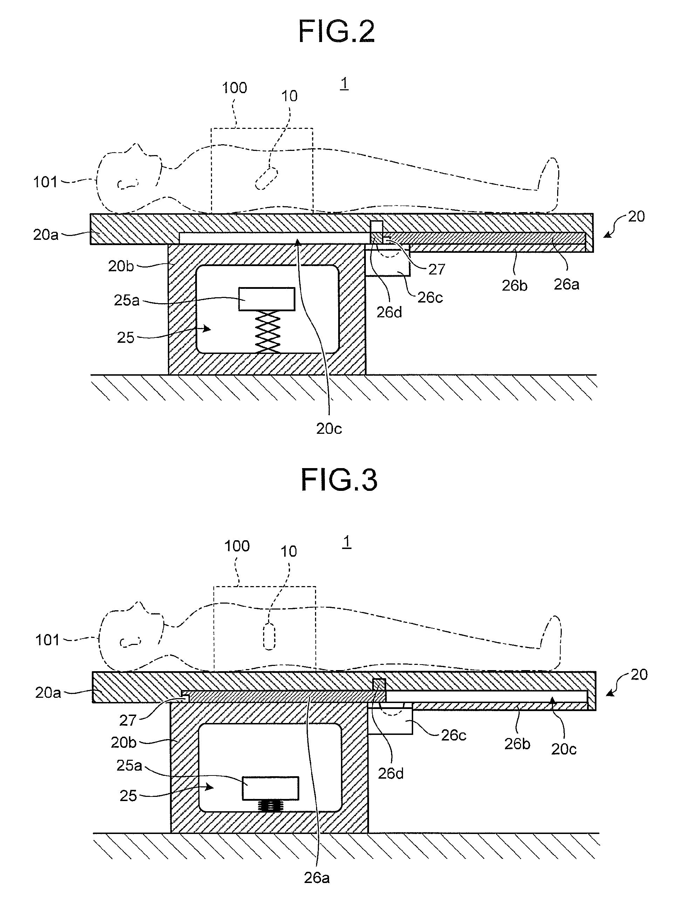

FIG. 2 and FIG. 3 are each a partial sectional side view schematically illustrating a configuration example of the guidance device 20. FIG. 2 illustrates a state in which a magnetic field for guiding the capsule endoscope 10 is not shielded (hereinafter, referred to as a magnetic field generating state). On the other hand, FIG. 3 illustrates a state in which a magnetic field for guiding the capsule endoscope 10 is shielded (hereinafter, referred to as a magnetic field shielding state).

As illustrated in FIG. 2 and FIG. 3, the guidance device 20 includes a bed 20a supported by a leg 20b. The bed 20a serves as a placing table on which a subject 101 is placed. Hereinafter, an area to which a magnetic field capable of guiding the capsule endoscope 10 is generated is referred to as an effective magnetic field area 100. In the first embodiment, the effective magnetic field area 100 is set in a portion of the region above the bed 20a. Usually, the subject 101 is placed on the bed 20a such that an examination (diagnosis) target region is overlapped with the effective magnetic field area 100.

The capsule endoscope 10 is introduced into the inside of the organ of the subject 101 along with a certain liquid through oral intake, then moves through the inside of the gastrointestinal tract, and is finally discharged to the outside of the subject 101. Meanwhile, the capsule endoscope 10 floats in a liquid introduced into the inside of the organ (for example, the inside of the stomach) of the subject 101, and sequentially takes an image of the inside of the subject 101 while being magnetically guided by the magnetic field generated by the guidance device 20. Then, the capsule endoscope 10 sequentially wirelessly transmits image information (image data) corresponding to an in-vivo image acquired by imaging. The detailed configuration of the capsule endoscope 10 will be described later.

The guidance device 20 includes: a receiver 21 that performs wireless communication with the capsule endoscope 10 to receive a wireless signal containing image information acquired by the capsule endoscope 10; a position detector 22 that detects a position of the capsule endoscope 10 within the subject 101 based on the wireless signal received from the capsule endoscope 10; a display unit 23a that acquires the image information from the wireless signal received by the receiver 21 and displays on a screen an in-vivo image obtained by performing specified signal processing on the image information as well as various information; a notifying unit 23b that performs notification to a user with visual information or auditory information; an operation input unit 24 that receives input of information that instructs various operations in the guidance system 1, and the like; a magnetic field generating unit 25 that generates a magnetic field for guiding the capsule endoscope 10; a shielding unit 26 that shields the magnetic field generated by the magnetic field generating unit 25; a shielding state detector 27 that detects a shielding state of a magnetic field by the shielding unit 26; a control unit 28 that controls these components; and a storage unit 29 that stores image information taken by the capsule endoscope 10, and the like.

The receiver 21 includes a plurality of antennas 21a, and sequentially receives a wireless signal from the capsule endoscope 10 through the plurality of antennas 21a. The receiver 21 selects an antenna having the highest received electric field strength among the plurality of antennas 21a, and performs, for example, demodulation processing on the wireless signal received from the capsule endoscope 10 through the selected antenna. Thus, the receiver 21 extracts image data on the inside of the subject 101 from this wireless signal. The receiver 21 outputs an image signal containing the extracted image data to the display unit 23a.

The position detector 22 performs calculation to estimate the position of the capsule endoscope 10 within the subject 101, based on the signal strength of the wireless signal received by the receiver 21.

The display unit 23a includes various displays such as a liquid crystal display, and generates an in-vivo image based on the image data input from the receiver 21 and a screen containing other various information, to be displayed on a display. Specifically, the display unit 23a, for example, sequentially displays on a screen a group of in-vivo images of the subject 101 taken by the capsule endoscope 10 while displaying the information on the position and posture of the capsule endoscope 10 and the information on guidance operation. At this time, the display unit 23a may display the position and posture of the capsule endoscope 10 estimated based on the magnetic field generated by the guidance device 20, or may display on a screen the position within the subject 101 corresponding to the displayed in-vivo image based on the position detection result of the position detector 22. Also, the display unit 23a displays, for example, a reduced image of the in-vivo image selected in accordance with control of the control unit 28, patient information and examination information of the subject 101, and the like. Furthermore, the display unit 23a displays on a screen a warning to a user and information such as a state of the guidance device 20 (for example, the magnetic field generating state or the magnetic field shielding state) in accordance with control of the control unit 28.

The notifying unit 23b includes, for example, an illumination device such as an LED and a voice device such as a buzzer, as well as a drive circuit that operates under control of the control unit 28 for controlling these devices. The notifying unit 23b notifies a user of a warning through the flashing of illumination or the sound of a buzzer, or notifies a user of a state of the guidance device 20 (for example, the magnetic field generating state or the magnetic field shielding state) through the lighting of illumination with a specified color.

The operation input unit 24 is achieved by an input device such as a joy stick, a console equipped with various buttons and various switches, and a keyboard, and receives input of various types of information such as the guidance instruction information for magnetically guiding the capsule endoscope 10 and the setting information for setting a specified mode to the guidance device 20. The guidance instruction information is information for controlling the position and posture of the capsule endoscope 10 that is a target of the magnetic guidance operation. In particular, the guidance instruction information includes information on the action of changing the position of the capsule endoscope 10 and the action of changing a tilt angle (an angle with respect to the vertical axis) of the capsule endoscope 10, and information on the action of changing an azimuth angle (an angle around the vertical axis) of the visual field (imaging units 11A and 11B described later) of the capsule endoscope 10. Hereinafter, the azimuth angle of the visual field is merely referred to as an azimuth angle. The operation input unit 24 inputs the above-described received information to the control unit 28.

The magnetic field generating unit 25 is disposed below the bed 20a (inside the leg 20b), and generates a magnetic field for changing the position, tilt angle and azimuth angle of the capsule endoscope 10 introduced into the subject 101 relative to the subject 101, to the effective magnetic field area 100. Here, in order to inhibit the leakage of the magnetic field generated by the magnetic field generating unit 25 into a space other than the effective magnetic field area 100 (for example, in a side surface direction of the leg 20b), the leg 20b is preferably made of a ferromagnetic substance such as an iron plate.

The magnetic field generating unit 25 has an extracorporeal permanent magnet 25a that generates a magnetic field, as well as a plane position changing unit 25b, a vertical position changing unit 25c, an elevation angle changing unit 25d, and a revolution angle changing unit 25e as a mechanism of translating and rotating the extracorporeal permanent magnet 25a.

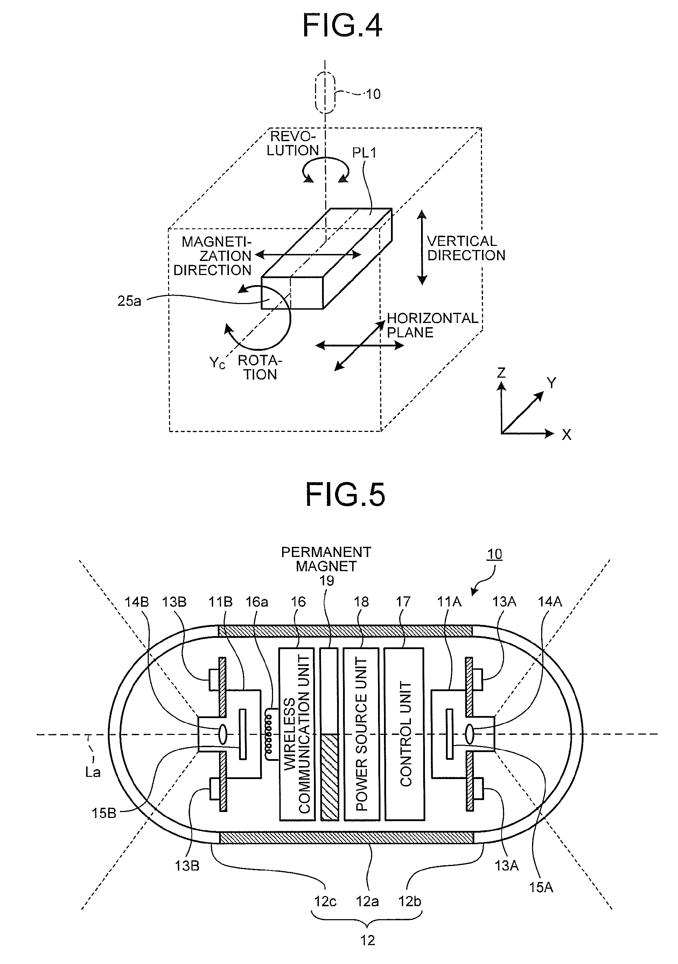

FIG. 4 is a schematic diagram for explaining an installed state of the extracorporeal permanent magnet 25a. As illustrated in FIG. 4, the extracorporeal permanent magnet 25a is achieved by, for example, a bar magnet having a rectangular parallelepiped shape. The extracorporeal permanent magnet 25a confines the capsule endoscope 10 within a region facing a plane that contains a magnetization direction and a first direction orthogonal to the magnetization direction. Hereinafter, the plane of the extracorporeal permanent magnet 25a that faces the capsule endoscope 10 is referred to as a capsule facing plane PL1 (a first plane).

The extracorporeal permanent magnet 25a is arranged using an optional plane parallel to the first direction (a y-axis direction in FIG. 4) as a reference. In the present first embodiment, the reference plane is set in parallel to a horizontal plane, and in an initial state of the capsule endoscope 10, the capsule facing plane PL1 is arranged so as to be parallel to the reference plane (a horizontal plane). Hereinafter, the arrangement of the extracorporeal permanent magnet 25a when the extracorporeal permanent magnet 25a is in an initial state of the capsule endoscope 10 is defined as an initial position. The magnetization direction at this time is defined as an x-axis direction; a direction within the horizontal plane orthogonal to the magnetization direction is defined as a y-axis direction; and a vertical direction is defined as a z-axis direction.

In the extracorporeal permanent magnet 25a, among the lengths of the sides in the three directions of the rectangular parallelepiped shape, the length of the side in the direction orthogonal to the magnetization direction on the capsule facing plane PL1 (the y-axis direction in FIG. 4) is longer than the lengths of the sides in the magnetization direction (the x-axis direction in FIG. 4) and in the direction orthogonal to the capsule facing plane PL1 (the z-axis direction in FIG. 4). Preferably, the extracorporeal permanent magnet 25a has a flat plate shape in which, among the lengths of the sides in the three directions of the rectangular parallelepiped shape, the length in the direction orthogonal to the capsule facing plane PL1 is the shortest. The shape of the extracorporeal permanent magnet 25a will be described in detail later.

The plane position changing unit 25b is a translation mechanism that translates the extracorporeal permanent magnet 25a on the horizontal plane set as a reference plane. That is, the movement is performed within the horizontal plane while maintaining a state in which the relative position of two magnetic poles magnetized in the extracorporeal permanent magnet 25a is ensured.

The vertical position changing unit 25c is a translation mechanism that translates the extracorporeal permanent magnet 25a in the vertical direction orthogonal to the horizontal plane set as a reference plane.

The elevation angle changing unit 25d is a rotation mechanism that rotates the extracorporeal permanent magnet 25a around an axis that is parallel to the capsule facing plane PL1 and orthogonal to the magnetization direction and that extends through the center of the extracorporeal permanent magnet 25a (hereinafter, referred to as a rotation axis $Y_C$), thereby to change the angle of the magnetization direction with respect to the horizontal plane set as a reference plane. Hereinafter, an angle formed between the extracorporeal permanent magnet 25a and the horizontal plane is defined as an elevation angle θ.

The revolution angle changing unit 25e rotates the extracorporeal permanent magnet 25a around an axis orthogonal to the reference plane. In the present first embodiment, the axis in the vertical direction extending through the center of the extracorporeal permanent magnet 25a is defined as a rotation axis of the extracorporeal permanent magnet 25a. Hereinafter, the rotation motion around the axis in the vertical direction of the extracorporeal permanent magnet 25a is referred to as a revolution motion. Also, the angle at which the extracorporeal permanent magnet 25a revolves with respect to the initial position is defined as a revolution angle ψ.

The revolution angle changing unit 25e revolves the extracorporeal permanent magnet 25a at the revolution angle ψ, to change the angle of the rotation axis $Y_C$ with respect to the initial position. In this state, the elevation angle changing unit 25d rotates the extracorporeal permanent magnet 25a around the rotation axis $Y_C$. This enables the azimuth angle and the tilt angle of the capsule endoscope 10 confined by the magnetic field generated by the extracorporeal permanent magnet 25a to be changed.

As illustrated in FIG. 2 and FIG. 3, the shielding unit 26 has: a plate-shaped magnetic body member 26a disposed on a lower surface of the bed 20a; a support 26b that slidably supports the magnetic body member 26a to a main surface of the bed 20a in a lower surface of the bed 20a; a drive unit 26c that drives the magnetic body member 26a along the bed 20a; and a fixing unit 26d.

Here, the extracorporeal permanent magnet 25a, unlike an electromagnet, cannot perform on/off of magnetic field formation, adjustment of magnetic field strength, and the like. That is, a permanent magnet constantly generates a magnetic field having certain strength. Therefore, when guidance operation of the capsule endoscope 10 is not performed, or when the guidance device 20 is not used, the magnetic field needs to be reduced in strength, or preferably shielded, in order to inhibit an unintended movement of the capsule endoscope 10 or an influence on the subject 101. In this regard, a configuration is conceivable in which when the guidance device 20 is not used, a covering unit made of a ferromagnetic substance covers the extracorporeal permanent magnet 25a for shielding. However, in this case, operation of shielding a magnetic field is complicated, and, for example, it is difficult to take a quick action in an emergency.

On the contrary, in the present first embodiment, the guidance device 20 is provided with the shielding unit 26. Thus, there is provided a configuration in which the magnetic field in the effective magnetic field area 100 can be shielded by a simpler operation.

The magnetic body member 26a is preferably made of a ferromagnetic substance, and is inserted below the bed 20a thereby to shield the magnetic field generated by the magnetic field generating unit 25 to the effective magnetic field area 100. The magnetic body member 26a has a material and a size (width × length × thickness) that allow the magnetic field generated by the magnetic field generating unit 25 to be shielded in the effective magnetic field area 100. As described herein, a width means a dimension in the width direction of the subject 101, and a length means a dimension in the body length direction of the subject 101. In the first embodiment, as the magnetic body member 26a, for example, there is used a member having a size of a width approximately equal to the width of the bed 20a × a length approximately half the length of the bed 20a.

A concave 20c to which the magnetic body member 26a is to be arranged is disposed on the lower surface of the bed 20a. The concave 20c is positioned so as to correspond to from the effective magnetic field area 100 that is a region on which the examination target region (for example, the stomach) of the subject 101 is placed, to a region on which the non-examination target region (for example, the leg) is placed. The magnetic body member 26a slides and moves in the concave 20c along the length direction of the bed 20a.

The support 26b supports the magnetic body member 26a arranged in the concave 20c upward. Preferably, in order to allow the magnetic body member 26a to easily slide, rails and pulleys may be disposed on an upper surface of the support 26b (a contact surface with the magnetic body member 26a).

The drive unit 26c drives the magnetic body member 26a to move in the concave 20c along the length direction of the bed 20a, thereby to insert and remove the magnetic body member 26a into and from between the magnetic field generating unit 25 and the effective magnetic field area 100. When the magnetic body member 26a is inserted between the magnetic field generating unit 25 and the effective magnetic field area 100, the guidance device 20 becomes in the magnetic field shielding state (see FIG. 3). On the other hand, when the magnetic body member 26a is removed from between the magnetic field generating unit 25 and the effective magnetic field area 100, the guidance device 20 becomes in the magnetic field generating state (see FIG. 2).

The fixing unit 26d is disposed near the middle of the concave 20c, and fixes the position of the magnetic body member 26a so that an unintended shifting between the magnetic field generating state and the magnetic field shielding state does not occur. Particularly, when the guidance device 20 is in the magnetic field generating state (see FIG. 2), the magnetic field generated by the magnetic field generating unit 25 causes a magnetic attracting force for sliding the magnetic body member 26a toward the position in the magnetic field shielding state, to act on the magnetic body member 26a. For this reason, the fixing unit 26d mechanically inhibits the movement of the magnetic body member 26a. Also, when shifting from the magnetic field generating state to the magnetic field shielding state is performed, a drive unit (not shown) that operates under control of the control unit 28 drives the fixing unit 26d to move upward in the drawing. Accordingly, the fixing state of the magnetic body member 26a is released (see FIG. 3). In this case, the fixing unit 26d can be manually operated to release the fixing state of the magnetic body member 26a.

The shielding state detector 27 is achieved by, for example, a pressure sensor that detects a pressure in the horizontal direction applied to the magnetic body member 26a. Here, as described above, when the guidance device 20 is in the magnetic field generating state (see FIG. 2), a magnetic attracting force acts between the extracorporeal permanent magnet 25a and the magnetic body member 26a. Therefore, at this time, the magnetic body member 26a is applied with a pressure in the left direction of the drawing in the horizontal direction. On the other hand, when the guidance device 20 is in the magnetic field shielding state (see FIG. 3), a magnetic attracting force between the extracorporeal permanent magnet 25a and the magnetic body member 26a mainly acts in an up-and-down direction. Therefore, the magnetic body member 26a is hardly applied with a pressure in the horizontal direction. Thus, a pressure in the horizontal direction applied to the magnetic body member 26a is detected by the shielding state detector 27, thereby to determine a state of the guidance device 20. That is, when the output value of the shielding state detector 27 is a specified threshold value or more, the guidance device 20 can be determined to be in the magnetic field generating state. Conversely, when the output value is lower than a specified threshold value, the guidance device 20 can be determined to be in the magnetic field shielding state. In this case, this determination is made by the control unit 28 based on the output result of the shielding state detector 27.

Also, as the shielding state detector 27, other than a pressure sensor, any sensor such as a compression sensor, a distortion sensor and an acceleration sensor (a force sensor) may be used as long as the magnitude of the force in a specified direction applied to the magnetic body member 26a can be detected. Alternatively, a force applied to the extracorporeal permanent magnet 25a, instead of the magnetic body member 26a, may be detected thereby to determine the state of the guidance device 20.

The control unit 28 controls action of each component of the magnetic field generating unit 25 based on the detection result of the position detector 22 and the guidance instruction information input from the operation input unit 24, to achieve the position, the tilt angle and the azimuth angle of the capsule endoscope 10 as a user desires. Also, the control unit 28 controls the shielding unit 26 in accordance with an operation signal input from the operation input unit 24, so that the guidance device 20 is shifted to a state corresponding to the status of a capsule endoscope examination (the magnetic field generating state or the magnetic field shielding state).

The storage unit 29 is achieved by using a storage medium that stores information in a rewritable manner, such as a flash memory and a hard disk. The storage unit 29 stores information such as various programs and various parameters used when the control unit 28 controls the components of the guidance device 20 as well as image data of a group of the in-vivo images of the subject 101 taken by the capsule endoscope 10.

Next, the detailed configuration of the capsule endoscope 10 will be described. FIG. 5 is a cross-sectional schematic diagram illustrating an example of an internal configuration of the capsule endoscope 10. As illustrated in FIG. 5, the capsule endoscope 10 includes a capsule-shaped casing 12 that is a sheath with a size allowing for being easily introduced into the organ of the subject 101, and imaging units 11A and 11B that take images of an imaging subject in different imaging directions from each other to generate image information. The capsule endoscope 10 also includes a wireless communication unit 16 that wirelessly transmits the image information acquired by the imaging units 11A and 11B to the outside, a control unit 17 that controls each component of the capsule endoscope 10, and a power source unit 18 that supplies electric power to each component of the capsule endoscope 10. The capsule endoscope 10 further includes a permanent magnet 19 for enabling magnetic guidance by the guidance device 20.

The capsule-shaped casing 12 is a sheath case with a size allowing for being introduced into the organ of the subject 101, and is achieved by closing the opening ends on both sides of a tubular casing 12a with dome-shaped casings 12b and 12c. The dome-shaped casings 12b and 12c are each a dome-shaped optical member that is transparent to light in a specified wavelength range such as visible light. Also, the tubular casing 12a is a colored casing that is substantially opaque to visible light. The capsule-shaped casing 12 constituted by the tubular casing 12a and the dome-shaped casings 12b and 12c liquid-tightly contains, as illustrated in FIG. 5, the imaging units 11A and 11B, the wireless communication unit 16, the control unit 17, the power source unit 18 and the permanent magnet 19 therein.

The imaging unit 11A has an illumination unit 13A such as an LED, an optical system 14A such as a condenser lens, and an imaging element 15A such as a CMOS image sensor or a CCD. The illumination unit 13A emits illumination light such as white light to the imaging visual field of the imaging element 15A, and illuminates an imaging subject within the imaging visual field through the dome-shaped casing 12b. The optical system 14A concentrates reflected light from this imaging visual field on the imaging surface of the imaging element 15A, and forms an imaging subject image in the imaging visual field. The imaging element 15A receives the light that is reflected from the imaging visual field and concentrated on the imaging surface, and performs photoelectric conversion of the received optical signal, to generate image information indicating a subject image in the imaging visual field, that is an in-vivo image of the subject 101.

The imaging unit 11B has, similarly to the imaging unit 11A, an illumination unit 13B such as an LED, an optical system 14B such as a condenser lens, and an imaging element 15B such as a CMOS image sensor or a CCD.

As illustrated in FIG. 5, when the capsule endoscope 10 is a twin-lens type capsule medical apparatus that takes images of the front and the back in a long axis La direction, the imaging units 11A and 11B are arranged so that each optical axis is substantially parallel to or substantially coincide with the long axis La that is the central axis in the longitudinal direction of the capsule-shaped casing 12, and so that both imaging visual fields are opposed to each other. That is, the imaging units 11A and 11B are mounted so that the imaging surfaces of the imaging elements 15A and 15B are orthogonal to the long axis La.

The wireless communication unit 16 includes an antenna 16a, and sequentially wirelessly transmits the above-described image information acquired by the imaging units 11A and 11B to the outside via the antenna 16a. Specifically, the wireless communication unit 16 acquires an image signal based on the image information generated by the imaging unit 11A or the imaging unit 11B from the control unit 17, and performs modulation processing on the image signal, to generate a wireless signal obtained by modulating the image signal. The wireless communication unit 16 transmits this wireless signal to the outside receiver 21 through the antenna 16a.

The control unit 17 controls actions of the imaging units 11A and 11B and the wireless communication unit 16, and also controls input and output of signals among these components. Specifically, the control unit 17 allows the imaging element 15A to take an image of an imaging subject within the imaging visual field illuminated by the illumination unit 13A, and allows the imaging element 15B to take an image of an imaging subject within the imaging visual field illuminated by the illumination unit 13B. Also, the control unit 17 has a signal processing function of generating an image signal. The control unit 17 acquires image information from the imaging elements 15A and 15B, and for every acquisition, performs a specified signal processing on this image information, to generate an image signal containing image data. Furthermore, the control unit 17 controls the wireless communication unit 16 to sequentially wirelessly transmit such an image signal to the outside in chronological order.

The power source unit 18 is an electric storage unit such as a button-type battery or a capacitor, and has a switch unit such as a magnetic switch and an optical switch. The power source unit 18 switches an on/off state of the power source by the magnetic field applied from the outside. In an on-state, the electric power in the electric storage unit is appropriately supplied to each component of the capsule endoscope 10 (the imaging units 11A and 11B, the wireless communication unit 16, and the control unit 17). Also, in an off-state, the power source unit 18 stops the supply of electric power to each component of the capsule endoscope 10.

The permanent magnet 19 is provided for enabling magnetic guidance of the capsule endoscope 10 in the effective magnetic field area 100 with the magnetic field generated by the magnetic field generating unit 25, and is fixed and arranged to the inside of the capsule-shaped casing 12 so that the magnetization direction is tilted with respect to the long axis La. Specifically, the permanent magnet 19 is arranged so that the magnetization direction is orthogonal to the long axis La. The permanent magnet 19 acts while following the magnetic field applied from the outside. As a result, magnetic guidance of the capsule endoscope 10 by the magnetic field generating unit 25 is achieved.

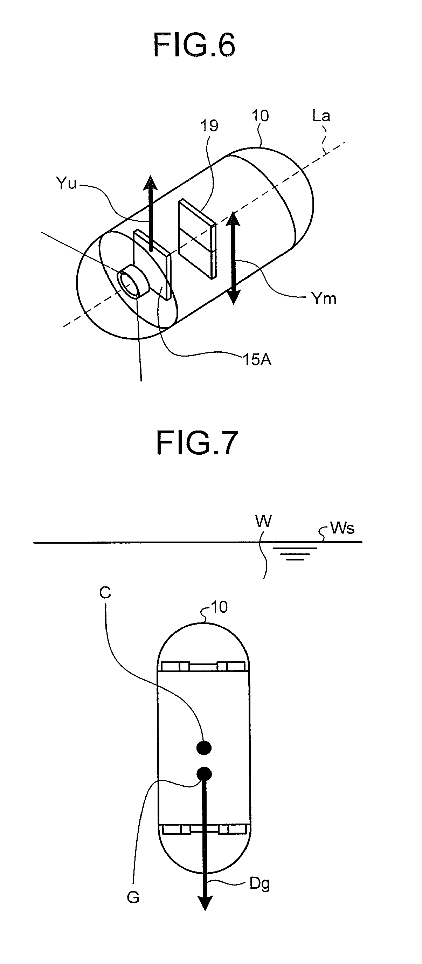

Here, with reference to FIG. 6, the relative positional relationship between the imaging elements 15A and 15B and the permanent magnet 19 will be described. The permanent magnet 19 is fixed and arranged inside the capsule-shaped casing 12 in a state of being relatively fixed with respect to the above-described imaging units 11A and 11B. More particularly, the permanent magnet 19 is arranged so that the magnetization direction thereof is relatively fixed with respect to the up-and-down direction of the imaging surface of each of the imaging elements 15A and 15B. Specifically, as illustrated in FIG. 6, the permanent magnet 19 is arranged so that a magnetization direction Ym thereof becomes parallel to an up-and-down direction Yu of the imaging surfaces of the imaging elements 15A and 15B.

FIG. 7 is a conceptual diagram for explaining a situation of the capsule endoscope 10 when a liquid W is introduced into the subject 101. Here, FIG. 7 illustrates a state in which the magnetic field for controlling the position, the tilt angle and the azimuth angle of the capsule endoscope 10 does not act on the permanent magnet 19.

The capsule endoscope 10 exemplified in the first embodiment is designed so that the specific gravity to the liquid W becomes nearly 1. Also, a gravity center G of the capsule endoscope 10 is set so as to be positioned off a geometric center C of the capsule endoscope 10 along the long axis La of the capsule endoscope 10 (the central axis in the longitudinal direction of the capsule endoscope 10: see FIG. 5). Specifically, the gravity center G of the capsule endoscope 10 is set at a position that is on the long axis La and off the geometric center C of the capsule-shaped casing 12 toward the imaging unit 11B side by adjusting the position of the components such as the power source unit 18 and the permanent magnet 19. Accordingly, the capsule endoscope 10 floats in the liquid W in a state where the long axis La thereof becomes substantially parallel to the vertical direction (that is, the gravity direction). In other words, the capsule endoscope 10 floats in the liquid W in a state where the line connecting the geometric center C and the gravity center G is upright. In such an upright posture, the capsule endoscope 10 directs the imaging visual field of the imaging unit 11A vertically upward, while directing the imaging visual field of the imaging unit 11B vertically downward. Here, the liquid W is a liquid that is harmless to the human body, such as water and a physiological salt solution.

Also, as described above, the permanent magnet 19 is arranged such that the magnetization direction Ym thereof (see FIG. 6) is orthogonal to the long axis La. That is, the magnetization direction Ym of the permanent magnet 19 coincides with the radial direction of the capsule endoscope 10. Therefore, when the magnetic field for controlling the position, the tilt angle and the azimuth angle of the capsule endoscope 10 does not act on the permanent magnet 19, the capsule endoscope 10 floats in the liquid W in a state where the magnetization direction Ym coincides with the horizontal direction. Also, at this time, the plane which passes the magnetization direction Ym and the line connecting the geometric center C and the gravity center G of the capsule-shaped casing 12 comes to be a vertical plane.

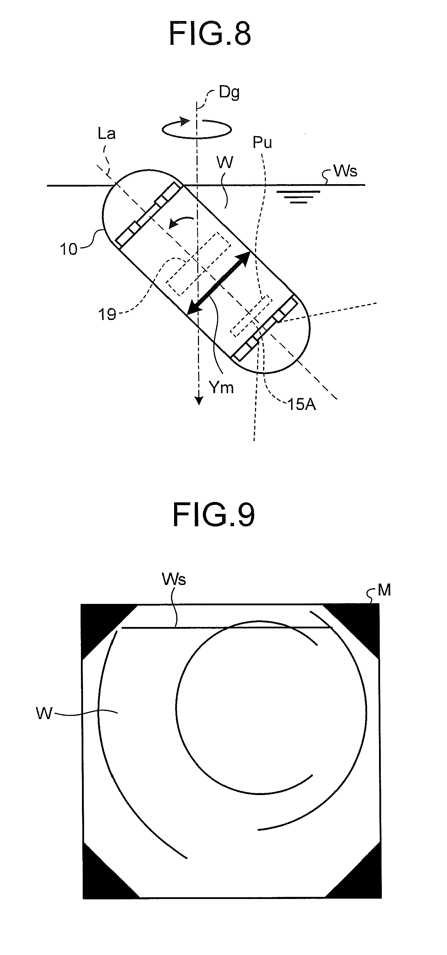

FIG. 8 is a conceptual diagram for explaining a situation of the capsule endoscope 10 in a state where the liquid W is introduced into the subject 101, and illustrates a state in which the magnetic field for controlling the tilt angle and the azimuth angle of the capsule endoscope 10 acts on the permanent magnet 19.

As illustrated in FIG. 8, the tilt of the long axis La of the capsule endoscope 10 to a gravity direction Dg can be controlled by permitting the magnetic field to act on the permanent magnet 19 of the capsule endoscope 10 from the outside. For example, by permitting the magnetic field having a magnetic force line direction that has an angle with respect to the horizontal plane to act on the permanent magnet 19, the capsule endoscope 10 can be tilted with respect to the gravity direction Dg so that the magnetization direction Ym of the permanent magnet 19 is substantially parallel to this magnetic force line. In this case, the tilt angle of the capsule endoscope 10 changes while the magnetization direction Ym maintains the state of being contained in the vertical plane. The magnetic field that performs such control is achieved when the elevation angle changing unit 25d of the guidance device 20 rotates the extracorporeal permanent magnet 25a (see FIG. 1 and FIG. 4).

Therefore, by applying the magnetic field that revolves around the gravity direction Dg with the capsule endoscope 10 tilted so that the capsule endoscope 10 revolves around the gravity direction Dg as indicated by an arrow, in-vivo images around the capsule endoscope 10 can be easily acquired. The magnetic field that performs such control is achieved when the revolution angle changing unit 25e of the guidance device 20 revolves the extracorporeal permanent magnet 25a (see FIG. 1 and FIG. 4).

At this time, the display unit 23a of the guidance device 20 displays an in-vivo image of the subject 101 by the capsule endoscope 10, in a display mode in which the up-and-down direction of the imaging subject in the in-vivo image associated with the magnetic guidance of the capsule endoscope 10 is the same as the up-and-down direction of the display screen. As a result, as illustrated in FIG. 9, on a display screen M of the display unit 23a, a liquid surface Ws imaged by an element in an upper region Pu of the imaging element 15A of the capsule endoscope 10 is displayed in the upper portion of the image corresponding to the imaging unit 11A. Furthermore, since the magnetization direction Ym of the permanent magnet 19 is parallel to the up-and-down direction Yu of the imaging surfaces of the imaging elements 15A and 15B, the direction parallel to the magnetization direction Ym of the permanent magnet 19 coincides with the up-and-down direction of the display screen of the display unit 23a.

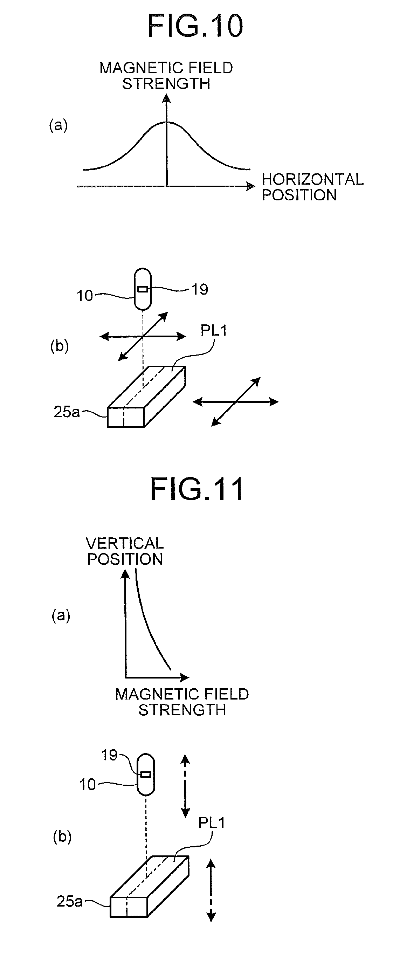

As illustrated in FIG. 10, the translational movement in the horizontal direction of the capsule endoscope 10 can be controlled by permitting a magnetic field having a peak of the magnetic field strength in the direction vertical to the capsule facing plane PL1 (see FIG. 10(a)) to act on the permanent magnet 19 of the capsule endoscope 10 so as to attract the permanent magnet 19 to the peak position of the magnetic field to confine the capsule endoscope 10 (see FIG. 10(b)). Specifically, such a magnetic field is achieved when the plane position changing unit 25b of the guidance device 20 moves the extracorporeal permanent magnet 25a within the horizontal plane.

As illustrated in FIG. 11, the translational movement in the vertical direction of the capsule endoscope 10 can be controlled by permitting a magnetic field having a distribution of the magnetic field strength that changes in accordance to the distance in the direction orthogonal to the capsule facing plane PL1 to acts on the permanent magnet 19 of the capsule endoscope 10. Specifically, such a magnetic field is achieved when the vertical position changing unit 25c of the guidance device 20 moves the extracorporeal permanent magnet 25a in the vertical direction.

For example, as illustrated in FIG. 11(a), when the capsule facing plane PL1 is set to be horizontal, a magnetic field having a magnetic strength that becomes weaker as the vertical position is higher acts on the permanent magnet 19. At this time, as illustrated in FIG. 11(b), when the extracorporeal permanent magnet 25a is moved upward to relatively lower the vertical position of the permanent magnet 19, the magnetic attracting force applied to the permanent magnet 19 becomes stronger so that the capsule endoscope 10 is biased downward. Here, the position in the vertical direction of the capsule endoscope 10 is nearly maintained at a position where the buoyancy of the capsule endoscope 10 to the liquid W, the gravity applied to the capsule endoscope 10, and the magnetic attracting force applied by the extracorporeal permanent magnet 25a are balanced.

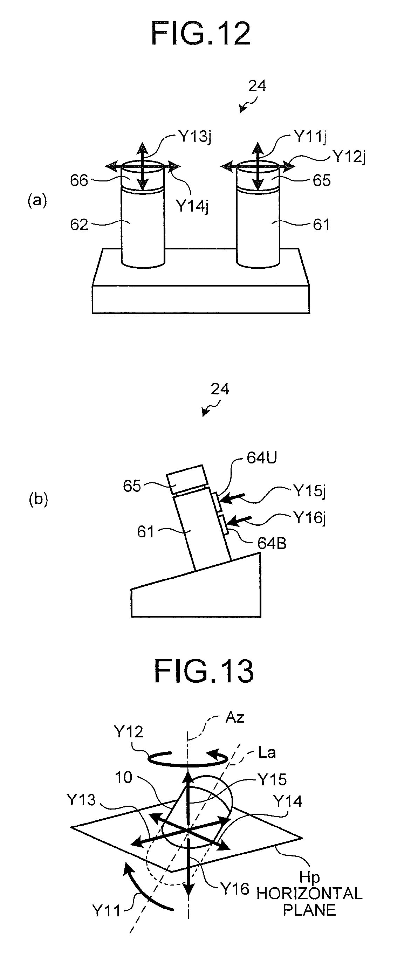

Next, the specific configuration and action of the operation input unit 24 illustrated in FIG. 1 will be described. FIG. 12(a) is a front view of the operation input unit 24, and FIG. 12(b) is a right side view of the operation input unit 24. FIG. 13 is a diagram illustrating a motion of the capsule endoscope 10 that is instructed by an operation of each component of the operation input unit 24.

As illustrated in FIG. 12(a), the operation input unit 24 includes two joy sticks 61 and 62 for three-dimensionally operating the magnetic guidance of the capsule endoscope 10 by the magnetic field generating unit 25. The joy sticks 61 and 62 each can perform a tilting operation in the up-and-down direction and the left-and-right direction.

As illustrated in FIG. 12(b), an up button 64U and a down button 64B are disposed on the back surface of the joy stick 61. The up button 64U is pressed to input guidance instruction information instructing the upward guidance of the capsule endoscope 10 to the control unit 28, and the down button 64B is pressed to input guidance instruction information instructing the downward guidance of the capsule endoscope 10 to the control unit 28. A capture button 65 is disposed on the top of the joy stick 61. The capture button 65 is pressed to capture an in-vivo image displayed on the display unit 23a. Also, an approach button 66 is disposed on the top of the joy stick 62. The approach button 66 is pressed to input guidance instruction information for guiding the capsule endoscope 10 so that the imaging unit 11A side of the capsule endoscope 10 is closer to the imaging object of the imaging unit 11A, to the control unit 28.

As illustrated in FIG. 12(a), the tilting direction in the up-and-down direction indicated by an arrow Y11j of the joy stick 61 corresponds to a tilting guidance direction in which the distal end of the capsule endoscope 10 swing so as to pass through a vertical axis Az as indicated by an arrow Y11 of FIG. 13. When the guidance instruction information corresponding to the tilting operation of the arrow Y11j of the joy stick 61 is input from the operation input unit 24 to the control unit 28, the control unit 28 calculates, based on this guidance instruction information, the guidance direction on the absolute coordinate system for the distal end of the capsule endoscope 10 in accordance with the tilting direction of the joy stick 61, and calculates a guidance amount in accordance with the tilting operation of the joy stick 61. Then, the magnetic field generating unit 25, for example, controls the elevation angle changing unit 25d to rotate the extracorporeal permanent magnet 25a in the calculated guidance direction in accordance with the calculated guidance amount.

As illustrated in FIG. 12(a), the tilting direction in the left-and-right direction indicated by an arrow Y12j of the joy stick 61 corresponds to a rotation guidance direction in which the capsule endoscope 10 rotates around the vertical axis Az as indicated by an arrow Y12 of FIG. 13. When the guidance instruction information corresponding to the tilting operation of the arrow Y12j of the joy stick 61 is input from the operation input unit 24 to the control unit 28, the control unit 28 calculates, based on this guidance instruction information, the guidance direction on the absolute coordinate system for the distal end of the capsule endoscope 10 in accordance with the tilting direction of the joy stick 61, and calculates the guidance amount in accordance with the tilting operation of the joy stick 61. Furthermore, the control unit 28, for example, controls the revolution angle changing unit 25e to revolve the extracorporeal permanent magnet 25a in the calculated guidance direction in accordance with the calculated guidance amount.

As illustrated in FIG. 12(a), the tilting direction in the up-and-down direction of the joy stick 62 as indicated by an arrow Y13j corresponds to a horizontal backward guidance direction or a horizontal forward guidance direction both proceeding in a direction in which the long axis La of the capsule endoscope 10 is projected on a horizontal plane Hp as indicated by an arrow Y13 of FIG. 13. When the guidance instruction information corresponding to the tilting operation of the arrow Y13j of the joy stick 62 is input from the operation input unit 24 to the control unit 28, the control unit 28 calculates, based on this guidance instruction information, the guidance amount and the guidance direction on the absolute coordinate system for the distal end of the capsule endoscope 10 in accordance with the tilting direction of the joy stick 62. Then, the control unit 28 controls the plane position changing unit 25b to translate the extracorporeal permanent magnet 25a in accordance with the calculated guidance direction and guidance amount.

As illustrated in FIG. 12(a), the tilting direction in the left-and-right direction of the joy stick 62 as indicated by an arrow Y14j corresponds to a horizontal right guidance direction or a horizontal left guidance direction in which the capsule endoscope 10 proceeds on the horizontal plane Hp vertically to a direction in which the long axis La is projected on the horizontal plane Hp as indicated by an arrow Y14 of FIG. 13. When the guidance instruction information corresponding to the tilting operation of the arrow Y14j of the joy stick 62 is input from the operation input unit 24 to the control unit 28, the control unit 28 calculates, based on this guidance instruction information, the guidance amount and the guidance direction on the absolute coordinate system for the distal end of the capsule endoscope 10 in accordance with the tilting direction of the joy stick 62. Then, the control unit 28 controls the plane position changing unit 25b to translate the extracorporeal permanent magnet 25a in accordance with the calculated guidance direction and guidance amount.

Also, the up button 64U and the down button 64B are disposed on the back surface of the joy stick 61. As indicated by an arrow Y15j of FIG. 12(b), when the up button 64U is pressed, an up action of proceeding upward as indicated by an arrow Y15 along the vertical axis Az illustrated in FIG. 13 is instructed. Also, as indicated by an arrow Y16j of FIG. 12(b), when the down button 64B is pressed, a down action of proceeding downward as indicated by an arrow Y16 along the vertical axis Az illustrated in FIG. 13 is instructed. When the guidance instruction information corresponding to the pressing operation of the arrow Y15j or Y16j of the up button 64U or the down button 64B is input from the operation input unit 24 to the control unit 28, the control unit 28 calculates, based on this guidance instruction information, the guidance amount and the guidance direction on the absolute coordinate system for the distal end of the capsule endoscope 10 in accordance with the pressed button. Then, the control unit 28 controls the vertical position changing unit 25c to translate the extracorporeal permanent magnet 25a in the vertical direction in accordance with the calculated guidance direction and guidance amount. For example, when the up button 64U is pressed, the vertical position changing unit 25c translates the extracorporeal permanent magnet 25a toward the lower direction of the vertical axis Az (the direction away from the capsule endoscope 10). Accordingly, the capsule endoscope 10 moves upward as indicated by the arrow Y15. On the other hand, when the down button 64B is pressed, the vertical position changing unit 25c translates the extracorporeal permanent magnet 25a toward the upper direction of the vertical axis Az (the direction closer to the capsule endoscope 10). Accordingly, the capsule endoscope 10 moves downward as indicated by the arrow Y16.

Here, the operation input unit 24 may further have an input device including various operation buttons and keyboards as well as the above-described joy sticks 61 and 62.

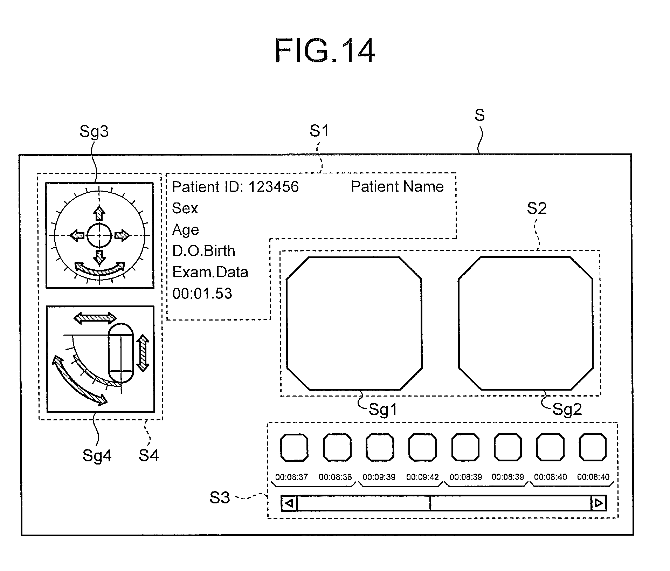

FIG. 14 is a schematic diagram illustrating a display example of a menu screen S displayed on the display unit 23a. On this menu screen S, various subject information such as patient name, patient ID, birth date, sex and age of the subject 101 is displayed in a region S1 in the upper left; a living body image Sg1 taken by the imaging unit 11A is displayed on the left side in a region S2 in the center; a living body image Sg2 taken by the imaging unit 11B is displayed on the right side; images captured by the pressing operation of the capture button 65 are displayed in a reduced manner along with capture times in a region S3 below the region S2; and a posture diagram Sg3 on the vertical plane and a posture diagram Sg4 on the horizontal plane are displayed as posture diagrams of the capsule endoscope 10 in a region S4 on the left side. The tilt angle and the azimuth angle of the capsule endoscope 10 displayed on the posture diagrams Sg3 and Sg4 indicate the tilt angle and the azimuth angle corresponding to the guidance instruction information of the operation input unit 24. In the first embodiment, since the input amount from the operation input unit 24 is reflected on a guiding force, the displayed tilt angle and azimuth angle of the capsule endoscope 10 can be considered as being substantially equal to the actual tilt angle and azimuth angle of the capsule endoscope 10, and the guidance instruction assistance to an operator is also improved. Here, the directions in which the capsule endoscope 10 can be guided are indicated by arrows in these posture diagrams Sg3 and Sg4. When operation of one of the guidance directions is input, the display color of the arrow corresponding to the input direction is changed to assist an operation by an operator.

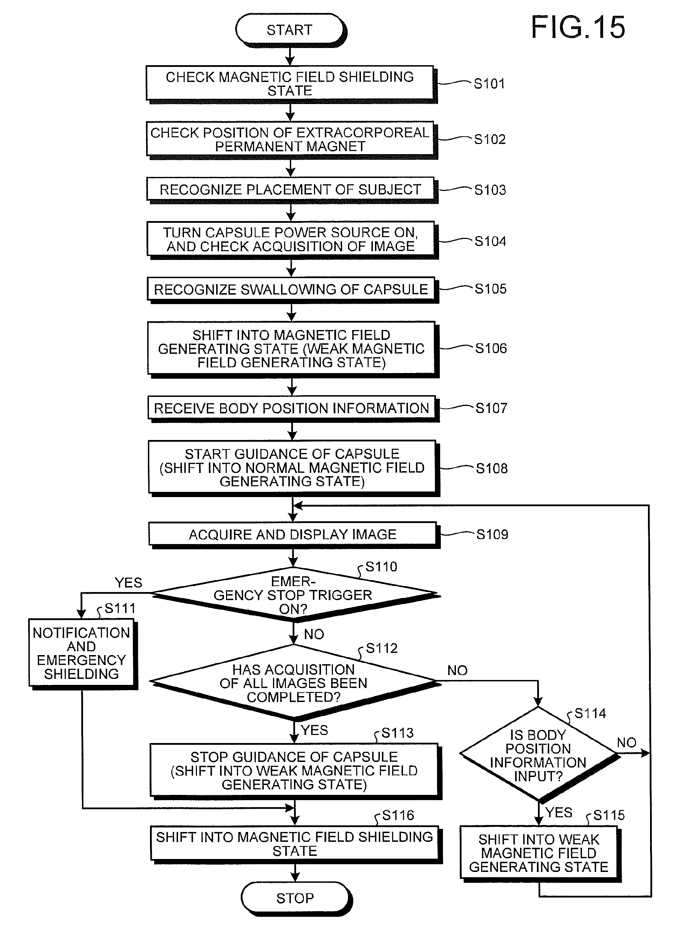

Next, the action of the guidance system 1 illustrated in FIG. 1 will be described. FIG. 15 is a flow chart illustrating the action of the guidance system 1.

When the capsule medical apparatus guidance system 1 is started, firstly, in step S101, the control unit 28 of the guidance device 20 checks, from the output result of the shielding state detector 27, that the guidance device 20 is in the magnetic field shielding state (see FIG. 3).

In subsequent step S102, the control unit 28 checks that the extracorporeal permanent magnet 25a is arranged at a position (an initial position) where the magnetic field strength is minimum in the effective magnetic field area 100. Here, when the extracorporeal permanent magnet 25a is not in the initial position, the control unit 28 controls the vertical position changing unit 25c to move the extracorporeal permanent magnet 25a to the initial position.

In step S103, when a signal indicating that the subject 101 has been placed on the bed 20a is input from the operation input unit 24, the control unit 28 recognizes that the subject 101 has been placed on the bed 20a. This signal may be input by a specified user operation (for example, pressing of a bed placement check button). Alternatively, the input may be performed when the bed 20a becomes in a specified state (for example, when a pressure sensor disposed on the subject placing surface of the bed 20a has an output value of higher than a specified threshold value).

In step S104, when the power source of the capsule endoscope 10 is turned on, the guidance device 20 receives a wireless signal transmitted from the capsule endoscope 10, and checks that an image taken by the capsule endoscope 10 can be acquired. Here, turning on the capsule endoscope 10 is achieved by turning on a magnetic switch or an optical switch of the power source unit 18 arranged in the capsule endoscope 10. That is, the magnetism or the light for activating the magnetic switch or the optical switch is applied from the outside.

In step S105, when a signal indicating that the capsule endoscope 10 has been swallowed by the subject 101 is input from the operation input unit 24, the control unit 28 recognizes that the capsule endoscope 10 has been swallowed by the subject 101. This signal may be input by a specified user operation (for example, pressing of a capsule swallowing check button). Alternatively, the signal may be input when image data transmitted from the capsule endoscope 10 becomes in a specified state (for example, in a specified state indicating that the color characteristic amount of an image is in the body).

In step S106, when a signal indicating the start of an examination is input from the operation input unit 24, the control unit 28 controls each component to shift the guidance device 20 to the magnetic field generating state. This signal indicating the start of an examination may be input by, for example, one action such as pressing of a specified button (for example, an examination start button) of the operation input unit 24, or an operation by the joy sticks 61 and 62, or the like.

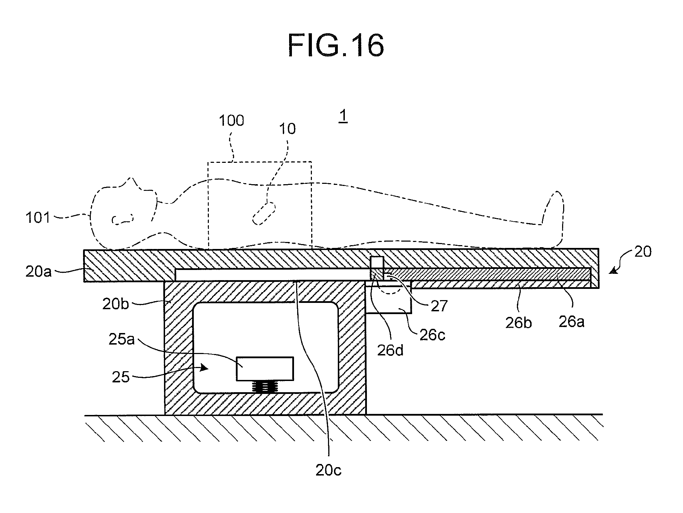

Accordingly, the magnetic body member 26a arranged between the magnetic field generating unit 25 and the effective magnetic field area 100 is removed, and the guidance device 20 becomes in the magnetic field generating state. At this time, since the magnetic field generating unit 25 is in the initial position (see step S102), the strength of the magnetic field to be generated to the effective magnetic field area 100 comes to be minimum. Accordingly, the magnetic field having a high strength can be inhibited from being rapidly applied to the capsule endoscope 10. Hereinafter, the magnetic field generating state in which the magnetic field generating unit 25 is in the initial position (see FIG. 16) is referred to as a weak magnetic field generating state.

In step S107, the control unit 28 receives information (for example, the body position information of the subject 101, such as decubitus left) input from the operation input unit 24, and causes the display device 23a to display the received information. At this time, the control unit 28 may acquire a relative coordinate system in the subject 101 based on the received information and the absolute coordinate system (the coordinate system based on the gravity direction) of the capsule endoscope 10, and perform calculation processing of estimating an observation direction and an observation region within the subject 101 based on the relative coordinate system.

In step S108, the control unit 28 starts guidance of the capsule endoscope 10, and controls the position, the elevation angle $\theta$ and the revolution angle $\psi$ of the extracorporeal permanent magnet 25a in accordance with guidance instruction information input from the operation input unit 24. Here, at this time, the extracorporeal permanent magnet 25a is moved upward higher than the initial position, so that the magnetic field having a higher strength comes to be formed to the effective magnetic field area 100. Accordingly, the guidance device 20 is shifted from the weak magnetic field generating state to the normal magnetic field generating state.

In step S109, the control unit 28 sequentially receives a wireless signal transmitted from the capsule endoscope 10, and controls an image taken by the capsule endoscope 10 to be displayed on the display unit 23a. A user operates the operation input unit 24 while referring to the image, thereby enabling the capsule endoscope 10 to be guided to a desired position, tilt angle and azimuth angle.

Here, a user may change the body position of the subject 101 as necessary. In this case, by performing a specified operation to the operation input unit 24, such as pressing of the operation button and input of the posture information through a keyboard or the like, a signal indicating that the body position of the subject 101 has been changed can be input into the guidance device 20. When the signal indicating that the body position of the subject 101 has been changed is input from the operation input unit 24, the control unit 28 controls the extracorporeal permanent magnet 25a to temporarily return to the initial position and become in a weak magnetic field generating state. Accordingly, the magnetic field having a high strength can be inhibited from being rapidly applied to the capsule endoscope 10 after the change of a body position.

In step S110, the control unit 28 determines whether or not an emergency stop trigger has been turned on. Here, the emergency stop trigger may be, for example, an emergency stop signal that is input from the operation input unit 24 in response to the pressing of a specified button (an emergency stop button), or may be a signal indicating that the placement of the subject 101 temporarily recognized in step S103 has come not to be recognized (for example, a decrease in the output value of the pressure sensor disposed on the subject placing surface). Besides, the detection of vibration caused by an earthquake or the like, or the rapid voltage drop in the guidance device 20 may be used as the emergency stop trigger.

When the emergency stop trigger has been turned on (step S110: Yes), the control unit 28 allows the display unit 23a and the notifying unit 23b to execute notification indicating that an emergency stop trigger has been turned on, and controls the shielding unit 26 so that the guidance device 20 is shifted to the magnetic field shielding state (emergency shielding) (step S111). As a specific notification method, a warning by visual information like "Emergency stop is executed" may be displayed on the display unit 23a; the notifying unit 23b may execute a warning by another visual information like the flashing of illumination or a warning by auditory information like voice or a warning sound; or both of these may be executed. Thereafter, the action of the guidance system 1 proceeds to step S116. On the other hand, when the emergency stop trigger is not turned on (step S110: No), the action of the guidance system 1 proceeds to step S112.

In step S112, the control unit 28 determines whether or not a signal indicating that the acquisition of all images has been completed has been input from the operation input unit 24. This signal may be input by a specified user operation (for example, pressing of an image acquisition completion button or a guidance stop button). Alternatively, the signal may be input when the number of images received from the capsule endoscope 10 has reached a specified amount, or when a specified time has elapsed after the power source of the capsule endoscope 10 was turned on.

When the acquisition of all images has been completed (step S112: Yes), the control unit 28 allows the action of the magnetic field generating unit 25 to be stopped so as to stop the guidance of the capsule endoscope 10. At the same time, the extracorporeal permanent magnet 25a is returned to the initial position to shift the guidance device 20 to the weak magnetic field generating state (step S113).

On the other hand, when all images have not yet acquired (step S112: No), the control unit 28 determines whether or not the body position information has been newly input from the operation input unit 24 (step S114). When the body position information has been newly input (step S114: Yes), the control unit 28 allows the extracorporeal permanent magnet 25a to return to the initial position to shift the guidance device 20 to the weak magnetic field generating state (step S115). Thereafter, the action of the guidance system 1 proceeds to step S109. On the other hand, when the body position information is not newly input (step S114: No), the action of the guidance system 1 directly proceeds to step S109.

In step S116, the control unit 28 controls the shielding unit 26 so that the guidance device 20 is shifted to the magnetic field shielding state. Accordingly, the action of the guidance system 1 is stopped. Thereafter, a user prompts the subject 101 to leave the bed 20a.

Here, although in the above description, the shifting between the magnetic field generating state and the magnetic field shielding state is performed by the shielding unit 26 under control of the control unit 28, a user may manually move the magnetic body member 26a to perform this shifting.

Next, the condition for the shape of the extracorporeal permanent magnet 25a will be described.

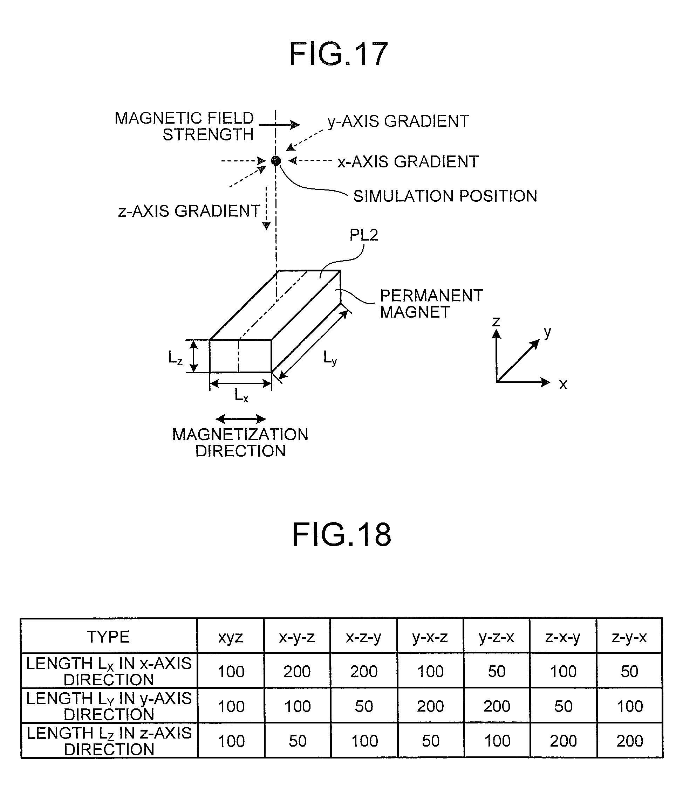

The inventors calculated the relationship between the shape (the ratio among length, width and height) of a permanent magnet and the generated magnetic field by simulation, in order to efficiently generate a magnetic field for guiding the capsule endoscope 10 from the extracorporeal permanent magnet 25a. FIG. 17 is a schematic diagram for explaining the evaluation items in this simulation. As illustrated in FIG. 17, in the present simulation, a magnetization direction of a permanent magnet was set in an x-axis direction; a direction orthogonal to the magnetization direction on the plane PL2 facing a simulation position was set in a y-axis direction; and a direction orthogonal to the plane PL2 was set in a z-axis direction. Then, the magnetic field strength in the simulation position, and the magnetic field gradients in the z-axis direction, the x-axis direction and the y-axis direction in the simulation position were evaluated. Here, the magnetic strength is involved in the guidance when changing the tilt angle and the azimuth angle of the capsule endoscope 10. The magnetic field gradient in the z-axis direction is involved in the guidance in the z-axis direction of the capsule endoscope 10. The magnetic field gradient in the x-axis direction is involved in the guidance in the x-axis direction of the capsule endoscope 10. The magnetic field gradient in the y-axis direction is involved in the guidance in the y-axis direction of the capsule endoscope 10.

Also, in the present simulation, a permanent magnet having a rectangular parallelepiped shape (including a cube) was used. FIG. 18 is a table illustrating the ratio in length among sides of a permanent magnet used in the simulation. As illustrated in FIG. 18, a "length $L_x$ in the x-axis direction" indicates a length $L_x$ of a side parallel to the x-axis; a "length $L_y$ in the y-axis direction" indicates a length $L_y$ of a side parallel to the y-axis; and a "length $L_z$ in the z-axis direction" indicates a length $L_z$ of a side parallel to the z-axis (see FIG. 17). Also, in the column of "type" in FIG. 18, the sides of each permanent magnet are indicated in a descending order of the length from the left. For example, type "x-y-z" indicates a rectangular parallelepiped shape in which a side parallel to the x-axis is the longest, and a side parallel to the z-axis is the shortest ($L_x > L_y > L_z$). Here, type "xyz" indicates a cube in which all sides have the same length ($L_x = L_y = L_z$).

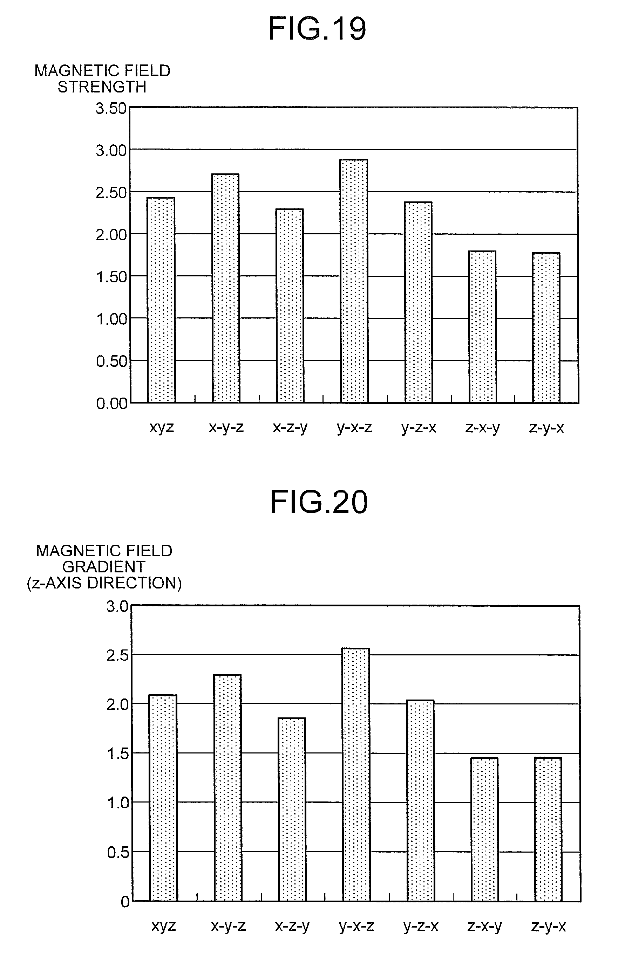

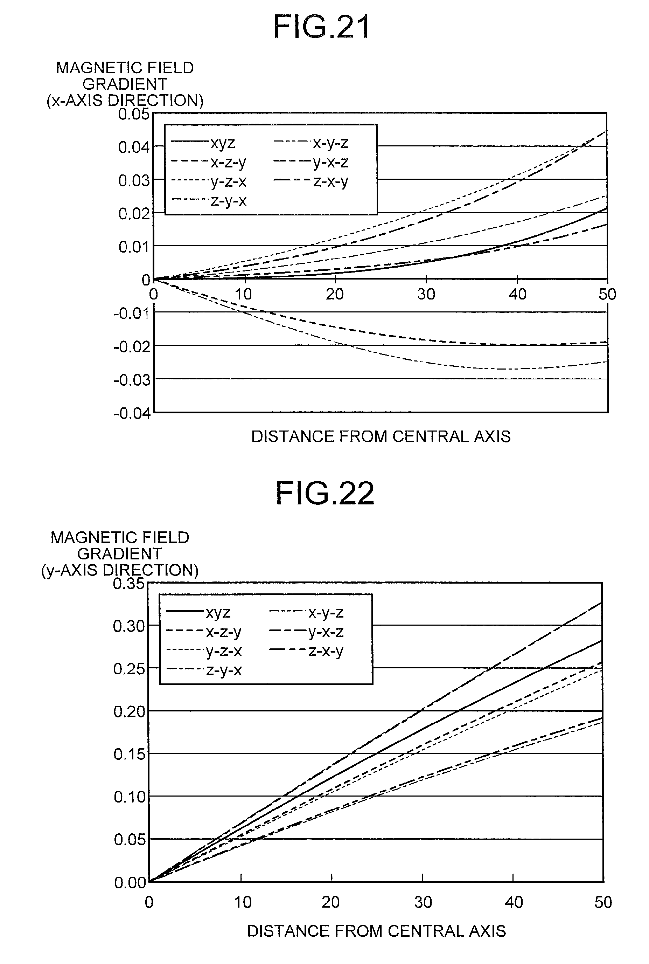

FIG. 19 is a graph illustrating magnetic field strengths of the permanent magnets illustrated in FIG. 18. FIG. 20 is a graph illustrating magnetic field gradients in the z-axis direction generated by the permanent magnets illustrated in FIG. 18. FIG. 21 is a graph illustrating magnetic field gradients in the x-axis direction generated by the permanent magnets illustrated in FIG. 18. FIG. 22 is a graph illustrating magnetic field gradients in the y-axis direction generated by the permanent magnets illustrated in FIG. 18. Here, in FIG. 19, the values of the magnetic field strengths are normalized. Also, in FIG. 20 to FIG. 22, the values of the magnetic field gradients are normalized. In FIG. 21 and FIG. 22, the horizontal axis indicates the normalized value of the distance from the axis (central axis) in the z-axis direction passing through the center of the permanent magnet.

In order to efficiently control the tilt angle and the azimuth angle of the capsule endoscope 10, the magnetic field strength generated by a permanent magnet is preferably high. In this regard, as illustrated in FIG. 19, a magnet with a relatively high magnetic field strength was type y-x-z and type x-y-z. Therefore, it can be seen that the shape suitable for the control of the tilt angle and the azimuth angle of the capsule endoscope 10 is a shape in which the length $L_z$ in the z-axis direction is shorter than the length $L_y$ in the y-axis direction. Furthermore, it can be said that a flat shape is more preferred in which the length $L_z$ in the z-axis direction is shorter than the length $L_x$ in the x-axis direction and the length $L_y$ in the y-axis direction.

Also, when changing the tilt angle of the capsule endoscope 10 (that is, when rotating a permanent magnet around the axis parallel to the y-axis), a smaller projected area on the zx plane orthogonal to the y-axis is preferred so that the movement region of the permanent magnet during rotation can be reduced. Therefore, the length of $L_x$ in the x-axis direction is favorably shorter. In this case, since the permanent magnet can be installed further closer to the subject 101, the magnetic field having a high strength can be efficiency generated within the subject 101, thereby enabling the magnetic field generating unit 25 to be miniaturized.

For performing position control in the vertical direction of the capsule endoscope 10, the magnetic field gradient in the vertical direction is preferably large. In this regard, as illustrated in FIG. 20, a magnet with a relatively high magnetic field gradient in the z-axis direction was type y-x-z and type x-y-z. Therefore, it can be seen that the shape suitable for the position control in the vertical direction of the capsule endoscope 10 is a flat shape having a shorter length $L_z$ in the z-axis direction.

For performing position control in the horizontal direction of the capsule endoscope 10, the magnetic field gradient in the horizontal direction is preferably large. In this regard, as illustrated in FIG. 21, a magnet with a relatively high magnetic field gradient in the x-axis direction was type y-x-z and type y-z-x. Here, it was found that in the cases of type x-z-y and type x-y-z, the peak of the magnetic field gradient is formed at a position away from the permanent magnet. Also, as illustrated in FIG. 22, a magnet with a relatively high magnetic field gradient in the y-axis direction was type y-x-z and type x-y-z. Thus, it can be seen that the shape suitable for the control in the horizontal direction of the capsule endoscope 10 is a shape in which the length $L_y$ in the y-axis direction is longer compared to the length $L_x$ in the x-axis direction and the length $L_z$ in the z-axis direction. Also, it can be said that the length $L_x$ in the x-axis direction is preferably not much longer compared to the length $L_y$ in the y-axis direction and the length $L_z$ in the z-axis direction.

From the results of the above-described simulation, it was found that the shape of the extracorporeal permanent magnet 25a suitable for the control of the capsule endoscope 10 is a flat plate shape in which the length in the y-axis direction is the longest while the length in the z-axis direction is the shortest. Then, the present inventors subsequently performed another simulation for calculating a suitable ratio among the lengths of the sides of the extracorporeal permanent magnet 25a.

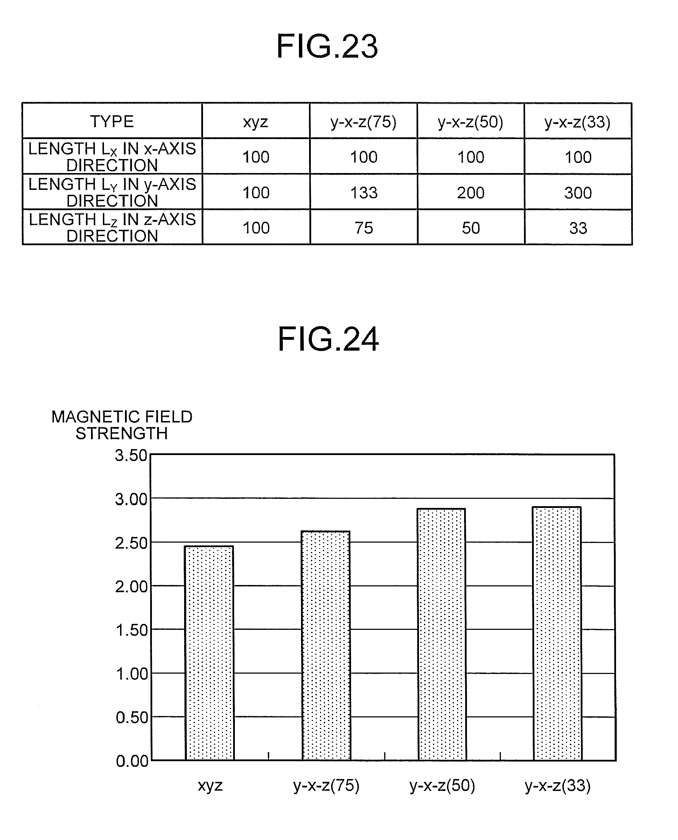

FIG. 23 is a table illustrating a ratio in length among sides of a permanent magnet used in another simulation. As illustrated in FIG. 23, a "length $L_x$ in the x-axis direction" corresponds to a length $L_x$ of a side parallel to the x-axis (a magnetization direction); a "length $L_y$ in the y-axis direction" corresponds to a length $L_y$ of a side parallel to the y-axis; and a "length $L_z$ in the z-axis direction" corresponds to a length $L_z$ of a side parallel to the z-axis (see FIG. 17). Also, in the column of "type" in FIG. 23, the sides of each permanent magnet are indicated in a descending order of the length from the left, and the numerical value in a parenthesis indicates the ratio of the length of the z-axis direction to the length of the x-axis direction. As illustrated in FIG. 23, in this simulation, all of the permanent magnets have a rectangular parallelepiped shape in which the side parallel to the y-axis direction is the longest while the side parallel to the z-axis direction is the shortest.

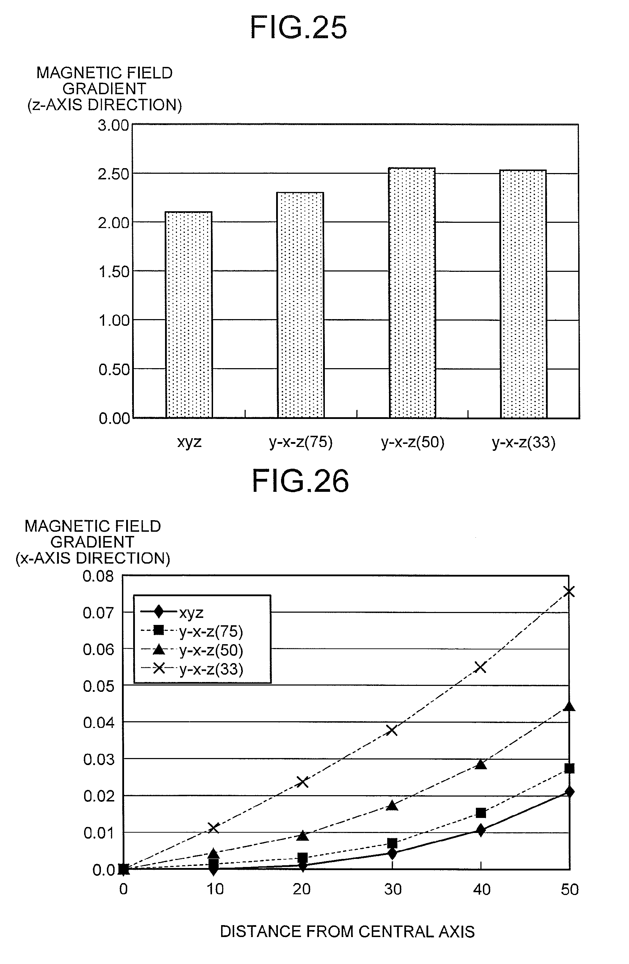

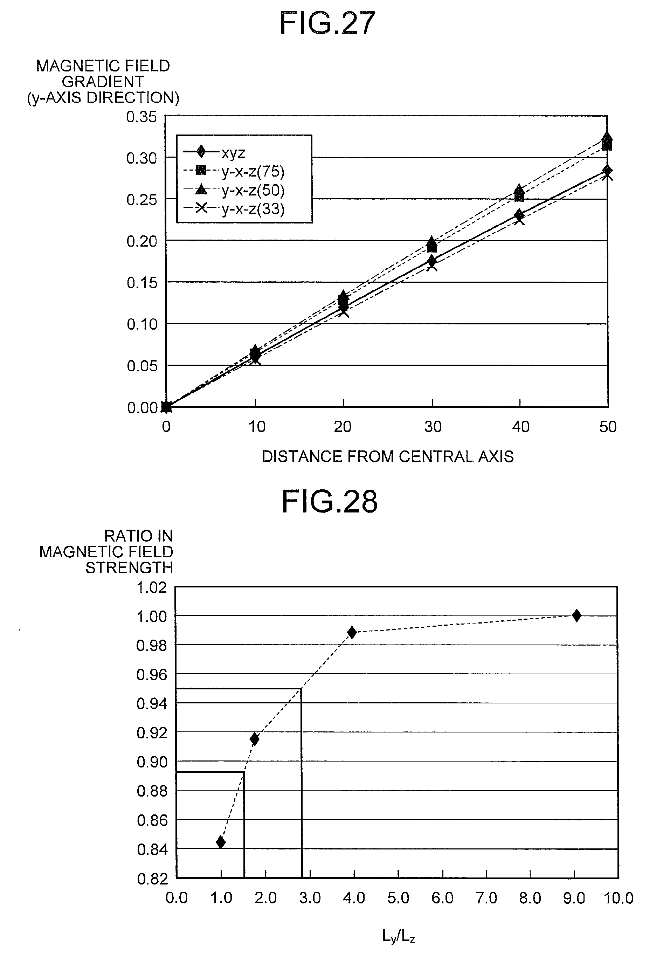

FIG. 24 is a graph illustrating magnetic field strengths of the permanent magnets illustrated in FIG. 23. FIG. 25 is a graph illustrating magnetic field gradients in the z-axis direction generated by the permanent magnets illustrated in FIG. 23. FIG. 26 is a graph illustrating magnetic field gradients in the x-axis direction generated by the permanent magnets illustrated in FIG. 23. FIG. 27 is a graph illustrating magnetic field gradients in the y-axis direction generated by the permanent magnets illustrated in FIG. 23. Here, in FIG. 24, the values of the magnetic field strengths are normalized. Also, in FIG. 25 to FIG. 27, the values of the magnetic field gradients are normalized. In FIG. 26 and FIG. 27, the horizontal axis indicates the normalized value of the distance from the axis (central axis) in the z-axis direction passing through the center of the permanent magnet.

As illustrated in FIG. 24 and FIG. 25, all of the permanent magnets had a favorable result in terms of the magnetic field strength and the magnetic field gradient in the z-axis direction. This result shows that the effect obtained by changing the ratio of the lengths of the sides of a permanent magnet is small.

On the other hand, as illustrated in FIG. 26, it can be seen that as the length $L_y$ in the y-axis direction is longer compared to the length $L_z$ in the z-axis direction of the permanent magnet (for example, type y-x-z(33) and type y-x-z(50)), the magnetic field gradient in the x-axis direction significantly improves. On the other hand, in this case, it can be seen that when this ratio is extreme (for example, type y-x-z(33)), as illustrated in FIG. 27, the magnetic field gradient in the y-axis direction deteriorates. However, since the value of the magnetic field gradient in the x-axis direction is smaller compared to that of the magnetic field gradient in the y-axis direction, the ratio between the length $L_y$ in the y-axis direction and the length $L_z$ in the z-axis direction may be determined in consideration of the balance among the magnetic field gradients in the axis directions.

FIG. 28 is a graph illustrating the relationship between the ratio $L_y/L_z$ of the length $L_y$ in the y-axis direction to the length $L_z$ in the z-axis direction, and the ratio of the magnetic field strength of the permanent magnet having each of the above-described ratios to the magnetic field strength of the permanent magnet of type y-x-z(33). As illustrated in FIG. 28, when the length $L_y$ in the y-axis direction to the length $L_z$ in the z-axis direction becomes 1.5 times, the magnetic field strength of approximately 90% of the magnetic field strength generated by the permanent magnet of type y-x-z(33) which is the permanent magnet having a sufficiently long length $L_y$ to the length of $L_z$ can be generated. Furthermore, when the length $L_y$ in the y-axis direction to the length $L_z$ in the z-axis direction becomes 3 times or more, the above-described ratio of the magnetic field strength comes to be 95%. Therefore, a preferred shape of the permanent magnet may be a shape in which the length $L_y$ in the y-axis direction to the length $L_z$ in the z-axis direction is 1.5 times or more, or 3 times or more.

Based on the above results of the simulation, the shape of the extracorporeal permanent magnet 25a was focused on a shape in which the length $L_y$ in the y-axis direction is the longest ($L_y > L_x$ and $L_y > L_z$). Then, further detailed simulation was conducted.

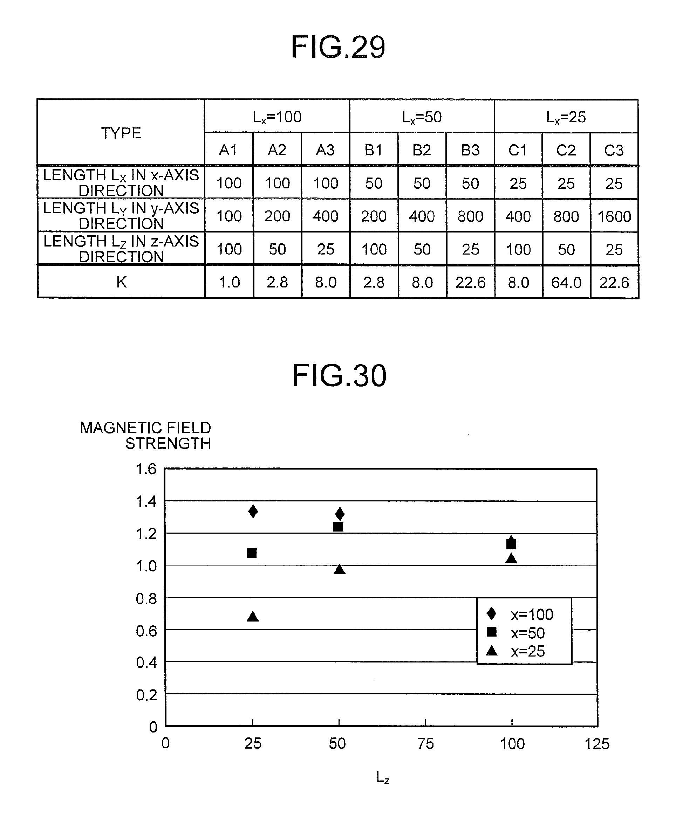

FIG. 29 is a table illustrating a ratio in length among the sides of the permanent magnet used in the present simulation. As illustrated in FIG. 29, a "length $L_x$ in the x-axis direction" corresponds to a length $L_x$ of a side parallel to the x-axis (the magnetization direction); a "length $L_y$ in the y-axis direction" corresponds to a length $L_y$ of a side parallel to the y-axis; and a "length $L_z$ in the z-axis direction" corresponds to a length $L_z$ of a side parallel to the z-axis (see FIG. 17). Also, as illustrated in FIG. 29, types A1 to A3 are indicated as having a length $L_x$ of 100; types B1 to B3 are indicated as having a length $L_x$ of 50; and types C1 to C3 are indicated as having a length $L_x$ of 25. Furthermore, a value K indicated in the bottom line of FIG. 29 is a value representing the characteristics of the shape of the permanent magnet, and is defined with the lengths $L_x$, Ly and $L_z$ as below:

$$K = \sqrt{\frac{L_y^2}{L_x \times L_z}} \ .$$

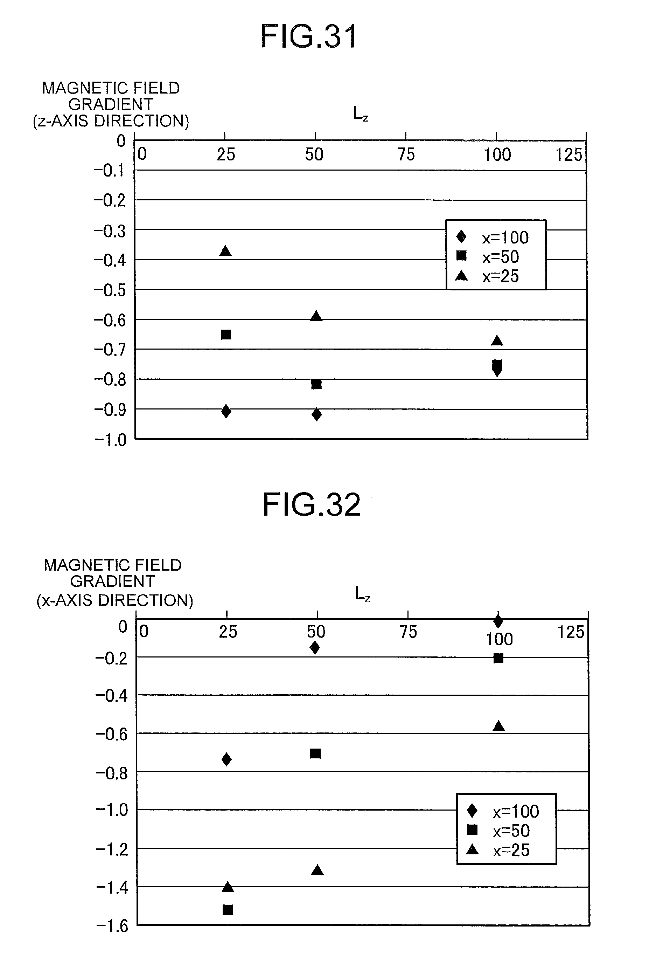

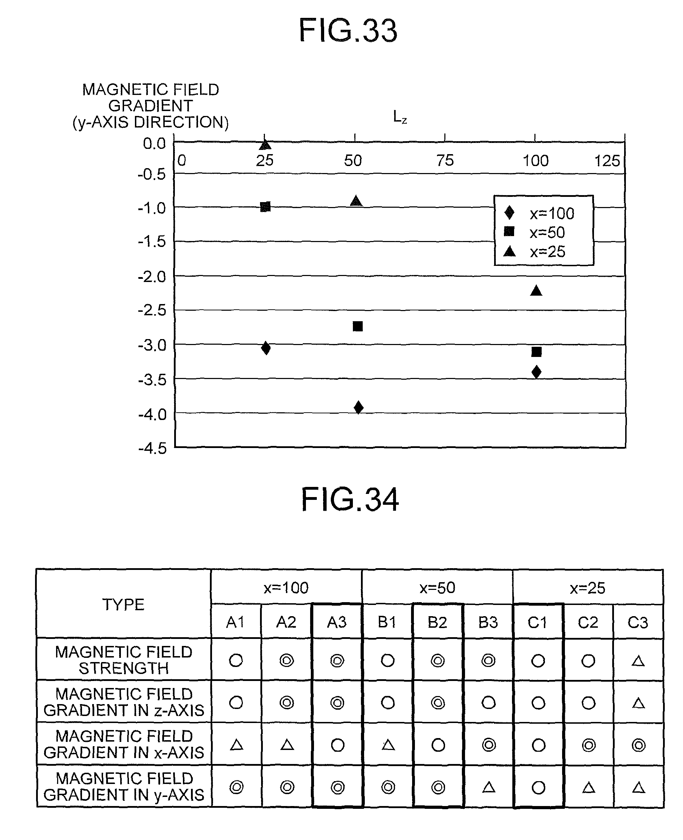

FIG. 30 is a graph illustrating a relationship between the length $L_z$ in the z-axis direction and the magnetic field strength of each of the permanent magnets illustrated in FIG. 29. FIG. 31 is a graph illustrating a relationship between the length $L_z$ of each of the permanent magnets illustrated in FIG. 29 and the magnetic field gradient in the z-axis direction. FIG. 32 is a graph illustrating a relationship between the length $L_z$ of each of the permanent magnets illustrated in FIG. 29 and the magnetic field gradient in the x-axis direction. FIG. 33 is a graph illustrating a relationship between the length $L_z$ of each of the permanent magnets illustrated in FIG. 29 and the magnetic field gradient in the y-axis direction. Here, in FIG. 30, the values of the magnetic field strengths are normalized. Also, in FIG. 31 to FIG. 33, the values of the magnetic field gradients are normalized.

FIG. 34 is a table illustrating the results indicated in FIG. 30 to FIG. 33, and the magnetic field strength or the magnetic field gradients in the x-axis, the y-axis and the z-axis are evaluated and classified into three ranks of large, moderate and small. Here, in FIG. 34, for each of the evaluation items, evaluation as large is indicated by a "double circle" symbol; evaluation as moderate is indicated by a "circle" symbol; and evaluation as small is indicated by a "triangle" symbol.

From FIG. 34, the permanent magnets of types A3, B2 and C1 do not contain "small (triangle)" in the evaluation results of all items, and have a favorable balance among the magnetic field strength and the magnetic field gradients in the x-axis, y-axis and z-axis. Thus, it can be said that a magnetic field is efficiently generated. Conversely, the permanent magnet of type C3 is evaluated as large (double circle) only in the magnetic field gradient of the x-axis direction, and is evaluated as small (triangle) in other items. Thus, it can be said that the generation efficiency of a magnetic field is significantly low. It can be said that the efficiency of the magnetic field caused by the permanent magnet of type (A1, A2, B1, B3 and C2) other than the above is between types A3, B2 and C1, and type C3.

From these results, it can be said that the shape of a permanent magnet that can efficiently generate a magnetic field has a K value higher than 1.0 and 22.6 or lower ($1 < K \leq 22.6$).

Also, in comparison among the permanent magnets of types A3, B2 and C1, when the length $L_x$ in the x-axis direction becomes shorter with respect to the length $L_z$ in the z-axis direction, the magnetic field strength, the magnetic field gradient in the z-axis direction, and the magnetic field gradient in the y-axis direction are decreased. Therefore, the length $L_x$ in the x-axis direction is preferably not much shorter compared to the length $L_z$ in the z-axis direction.

From the above, the condition for the aspect ratio for efficiently generating a magnetic field for guiding the capsule endoscope 10 from the extracorporeal permanent magnet 25a is as below. That is, the range of the K value may be $1 < K \leq 22.6$, and preferably, the K value may be around 8. Also, the length $L_x$ in the x-axis direction may be the length $L_z$ in the z-axis direction or longer ($L_x \geq L_z$).

As described above, according to the first embodiment, by using the extracorporeal permanent magnet 25a having the above-described condition, the guidance device 20 that can generate a magnetic field suitable for guiding the capsule endoscope 10 can be achieved.

Also, according to the first embodiment, since the shielding unit 26 that slides the magnetic body member 26a is disposed in the guidance device 20, a user can easily and quickly shift the guidance device 20 between the magnetic field generating state and the magnetic field shielding state by a simple input operation to the operation input unit 24 or manually.

Also, according to the first embodiment, since the state of the guidance device 20 is shifted by a simple operation in accordance with the status of the capsule endoscope examination, the examination can be safely performed. For instance, since the guidance device 20 is shifted to the magnetic field generating state after the start of an examination, a risk of unintentionally attracting a metal member to a magnetic field before and after the start of an examination can be inhibited. Also, for example, since the magnetic field strength is in the lowest state immediately after the shifting of the guidance device 20 to the magnetic field generating state, a risk of allowing a magnetic field having a high strength to be suddenly applied to the subject 101 can be prevented.

Also, according to the first embodiment, when the body position of the subject 101 is changed at the start of an examination or after the start of an examination, the extracorporeal permanent magnet 25a is returned to the initial position to shift the guidance device 20 to the weak magnetic field generating state. Accordingly, a situation can be prevented in which a magnetic field having a high strength is applied to the capsule endoscope 10 resulting in the movement of the capsule endoscope 10 to the position not expected by a user.

Also, according to the first embodiment, the capsule endoscope 10 is guided in a state where the capsule endoscope 10 floats in a liquid in which a liquid is introduced into the subject 101. For this reason, the magnetic field generating unit 25 for guiding the capsule endoscope 10 can be placed under the bed 20a on which the subject 101 is placed. Thus, the whole capsule medical apparatus guidance system can be miniaturized.

Here, although in the first embodiment described above, a pantoscopic capsule in which the imaging units 11A and 11B are disposed on the both sides of the capsule endoscope 10 is used, a monocular capsule in which an imaging unit is disposed on one end of the capsule endoscope may be used. In this case, by positioning the gravity center G of the capsule endoscope closer to the end on a side where the imaging unit is disposed, a capsule endoscope that takes only an image under a water surface (in water) can be achieved. On the other hand, by positioning the gravity center G of the capsule endoscope closer to the end on a side where the imaging unit is not disposed, a capsule endoscope that takes only a space above a water surface can be achieved.

Also, although in the first embodiment described above, the permanent magnet 19 is arranged such that the magnetization direction Ym is orthogonal to the long axis La of the capsule endoscope 10 (see FIG. 6), the permanent magnet 19 may be arranged such that the magnetization direction Ym coincides with the direction of the long axis La. At this time, the gravity center G may be positioned off the geometric center C of the capsule endoscope 10 in the radial direction. In this case, the posture of the capsule endoscope 10 can be uniquely controlled in the liquid W.

Also, in the first embodiment described above, in a state where a magnetic field is not applied, the gravity center G is positioned on the long axis La so that the capsule endoscope 10 floats with the long axis La directed in the vertical direction (see FIG. 7). However, the gravity center G may be positioned off the long axis La so that the capsule endoscope 10 floats with the long axis La tilted with respect to the vertical direction in a state where a magnetic field is not applied. In this case, the tilt angle and the azimuth angle of the capsule endoscope 10 can be uniquely controlled in the liquid W.

Alternatively, the gravity center G of the capsule endoscope 10 may be positioned off the geometric center C toward a direction different from the magnetization direction of the permanent magnet 19. In this case, the tilt angle and the azimuth angle of the capsule endoscope 10 can also be uniquely controlled in the liquid W.

Also, although in the first embodiment described above, a magnetic field for guiding the capsule endoscope 10 introduced into the subject 101 is generated by the magnetic field generating unit 25, a magnetic field that variously acts on the capsule endoscope 10 other than the above may be generated. For example, the magnetic field generating unit 25 may remotely perform on/off of a magnetic switch incorporated in the capsule endoscope 10.

Although in the first embodiment described above, the extracorporeal permanent magnet 25a included in the guidance device 20 has a rectangular parallelepiped shape, a permanent magnet having various shapes other than the rectangular parallelepiped shape, such as a polygonal column shape, a disk (or oval disk) shape, a frustum shape, or a shape similar to these may be employed as long as a permanent magnet whose length in the first direction orthogonal to the magnetization direction is longer than the length of the magnetization direction can be configured to confine the capsule endoscope 10 within a region facing the first plane parallel to the first direction. Preferably, the length in the second direction orthogonal to the magnetization direction of the extracorporeal permanent magnet and the first direction may be shorter than the length in the first direction. Even in a case where the shape of the extracorporeal permanent magnet is other than the rectangular parallelepiped shape, the details of, for example, the condition for the lengths of the magnetization direction as well as the first and second directions are similar to those described in the first embodiment. If a permanent magnet having a disk shape or an oval disk shape is used, the lengths in the magnetization direction as well as the first and second directions may be defined by the diameter, or the length of the major axis or the minor axis.

(Modified Example 1-1)

Next, modified example 1-1 of the first embodiment will be described.

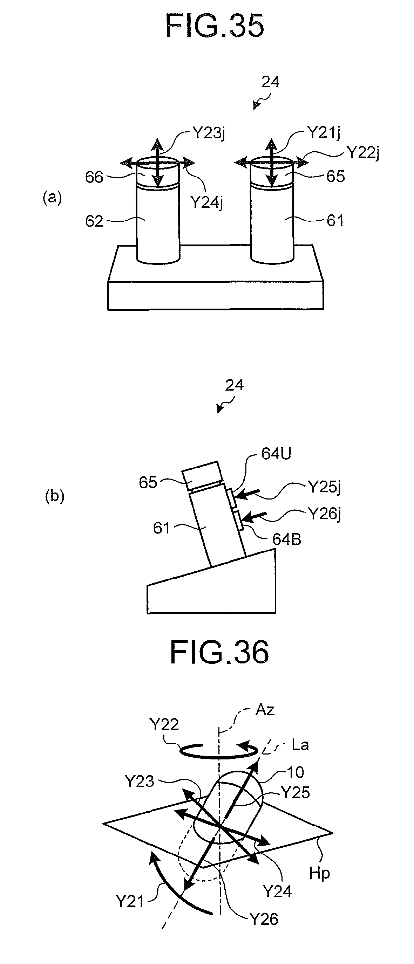

FIG. 35(a) is a front view of the operation input unit 24 according to modified example 1-1; FIG. 35(b) is a right side view of the operation input unit 24; and FIG.

36 is a diagram illustrating another example of the action content of the capsule endoscope 10 to be instructed by operation of each component of the operation input unit 24.

Each operation of the operation input unit 24 and guidance operation of the capsule endoscope 10 may be corresponded to each other so that, as described below, the capsule endoscope 10 can be guided along a plane orthogonal to a long axis La of the capsule endoscope 10, not along the horizontal plane Hp. Hereinafter, the movement of the capsule endoscope 10 corresponding to the guidance operation when guiding the capsule endoscope 10 along the plane orthogonal to the long axis La of the capsule endoscope 10 will be described.

As illustrated in FIG. 35(a), the tilting direction in the up-and-down direction indicated by an arrow Y23j of the joy stick 62 instructs, as illustrated in FIG. 36, a down guidance direction or an up guidance direction in which the capsule endoscope 10 proceeds as indicated by an arrow Y23 on a plane orthogonal to the long axis La. When the operation information corresponding to the tilting operation of the arrow Y23j of the joy stick 62 is input from the operation input unit 24 to the control unit 28, the magnetic field generating unit 25 calculates, based on this operation information, the guidance amount and the guidance direction on the absolute coordinate system for the distal end of the capsule endoscope 10 in accordance with the tilting direction of the joy stick 62. Then, the magnetic field generating unit 25 controls the plane position changing unit 25b and the vertical position changing unit 25c to translate the extracorporeal permanent magnet 25a in accordance with the calculated guidance direction and guidance amount.

As illustrated in FIG. 35(a), the tilting direction in the left-and-right direction indicated by an arrow Y24j of the joy stick 62 instructs, as illustrated in FIG. 36, a right guidance direction or a left guidance direction in which the capsule endoscope 10 proceeds as indicated by an arrow Y24 on a plane orthogonal to the long axis La. When the operation information corresponding to the tilting operation of the arrow Y24j of the joy stick 62 is input from the operation input unit 24 to the control unit 28, the control unit 28 calculates, based on this operation information, the guidance amount and the guidance direction on the absolute coordinate system for the distal end of the capsule endoscope 10 in accordance with the tilting direction of the joy stick 62. Then, the control unit 28 controls the plane position changing unit 25b to translate the extracorporeal permanent magnet 25a in accordance with the calculated guidance direction and guidance amount.

As illustrated in FIG. 35(b), the pressing of the up button 64U or the down button 64B as indicated by arrows Y25j and Y26j instructs, as illustrated in FIG. 36, a forward guidance direction or a backward guidance direction in which the capsule endoscope 10 proceeds forward or backward with respect to the imaging elements 15A and 15B as indicated by arrows Y25 and Y26 along the long axis La. When the operation information corresponding to the pressing operation of the arrow Y25j or Y26j of the up button 64U or the down button 64B is input from the operation input unit 24 to the control unit 28, the control unit 28 calculates, based on this operation information, the guidance amount and the guidance direction on the absolute coordinate system for the distal end of the capsule endoscope 10 in accordance with the pressed button. Then, the control unit 28 controls the plane position changing unit 25b and the vertical position changing unit 25c to translate the extracorporeal permanent magnet 25a in accordance with the calculated guidance direction and the calculation amount.

Here, as illustrated in FIG. 35(a), the tilting direction in the up-and-down direction indicated by an arrow Y21j of the joy stick 61 corresponds to a tilting guidance direction in which the distal end of the capsule endoscope 10 swings so as to pass through a vertical axis Az as indicated by an arrow Y21 of FIG. 36. The tilting direction in the left-and-right direction indicated by an arrow Y22j of the joy stick 61 corresponds to a rotation guidance direction in which the capsule endoscope 10 rotates around the vertical axis Az as indicated by an arrow Y22 of FIG. 36.

(Modified Example 1-2)

Next, modified example 1-2 of the first embodiment will be described.
The position detection of the capsule endoscope 10 within the subject 101 may be performed not only by the method based on the strength of the wireless signal received from the capsule endoscope 10 described in the first embodiment, but also by other various methods.
For example, a method may be used in which the position of the capsule endoscope 10 is detected based on acceleration applied to the capsule endoscope 10. In this case, an acceleration sensor that three-dimensionally detects the acceleration applied to the capsule endoscope 10 is disposed inside the capsule endoscope 10, and the detection result of the acceleration sensor is superimposed on a wireless signal to be transmitted at any time. The guidance device 20 integrates the accelerations applied to the capsule endoscope 10 based on the detection result of the acceleration sensor superimposed on the received wireless signal to obtain the relative change amount of the position of the capsule endoscope 10, and calculates the current position of the capsule endoscope 10 from this change amount.

(Modified Example 1-3)

Next, modified example 1-3 of the first embodiment will be described.
As the position detection method of the capsule endoscope 10 within the subject 101, a method of detecting an AC magnetic field may be used. In this case, an AC magnetic field generation unit that generates an AC magnetic field is disposed inside the capsule endoscope 10. On the other hand, a plurality of magnetic field sensors that detect an AC magnetic field is disposed on the guidance device 20 side.
The guidance device 20 detects an AC magnetic field generated by the capsule endoscope 10 with the plurality of magnetic field sensors that has been installed at a plurality of locations. Then, at least one of the position, the azimuth angle and the tilt angle of the capsule endoscope 10 is continuously calculated based on these detection results. In this case, the guidance device 20 may control the magnetic field generated by itself based on at least one of the calculated position, azimuth angle and tilt angle of the capsule endoscope 10. Also, the guidance device 20 may check whether or not the position of the capsule endoscope 10 is located within the measurement region in the subject 101 (within the region of the magnetic field generated by the magnetic field generating unit 25), and control the action of the shielding unit 26 based on this checking result. For example, when the capsule endoscope 10 is located within the measurement region in the subject 101, the control unit 28 controls the shielding unit 26 to remove the magnetic body member 26a from below the effective magnetic field area 100 so as to be in the magnetic field generating state. On the other hand, when the capsule endoscope 10 is located outside the measurement region within the subject 101, the control unit 28 controls the shielding unit 26 to insert the magnetic body member 26a below the effective magnetic field area 100 so as to be in the magnetic field shielding state.

(Modified Example 1-4)

Next, modified example 1-4 of the first embodiment will be described.
As the position detection method of the capsule endoscope 10 within the subject 101, another method of detecting an AC magnetic field will be described. In this case, an LC circuit that resonates with an AC magnetic field is disposed inside the capsule endoscope 10, and an AC magnetic field generation device and a plurality of magnetic field sensors that detects an AC magnetic field are disposed on the guidance device 20 side.
The guidance device 20 previously detects a first AC magnetic field that is generated by the AC magnetic field generation device, in a state where the capsule endoscope 10 is not located within the measurement region within the subject 101 (within the region of the magnetic field generated by the magnetic field generating unit 25). Then, when the capsule endoscope 10 is located within the measurement region within the subject 101, a second AC magnetic field containing the resonance magnetic field generated by the LC circuit in the capsule endoscope 10 is detected. Thereafter, the resonance magnetic field generated by the LC circuit in the capsule endoscope 10 is calculated from the difference value between the detected value of the first AC magnetic field and the detected value of the second AC magnetic field. The guidance device 20 continuously calculates the position coordinate of the capsule endoscope 10 in a three-dimensional space, based on the resonance magnetic field calculated as described above.

(Modified Example 1-5)

Next, modified example 1-5 of the first embodiment will be described.

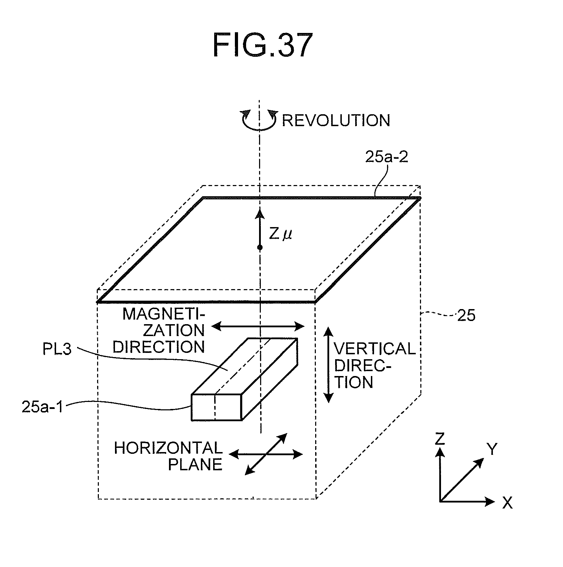

FIG. 37 is a schematic diagram illustrating a modified example of the magnetic field generating unit 25 illustrated in FIG. 1. A magnetism generation unit that generates magnetism in the magnetic field generating unit 25 is not limited to a configuration in which only the extracorporeal permanent magnet 25a is employed.

For example, as illustrated in FIG. 37, a magnetism generation unit may be achieved by an electromagnet having an extracorporeal permanent magnet 25a-1 and a coil 25a-2. The extracorporeal permanent magnet 25a-1 is arranged such that one plane (a capsule facing plane PL3) among four planes parallel to the magnetization direction thereof becomes parallel to the horizontal plane.

On the other hand, the coil 25a-2 is fixed to the guidance device 20 and arranged so that an orientation $Z\mu$ of a magnetic field generated by the coil 25a-2 becomes in the vertical direction. The coil 25a-2 generates a magnetic field in the vertical direction within the guidance region of the capsule endoscope 10. The generated magnetic field has uniformity higher than by the extracorporeal permanent magnet 25a-1. Also, the magnetic field strength thereof can be controlled by the control unit 28.

In this case, the tilt angle of the capsule endoscope 10 is controlled by a synthesis magnetic field of the magnetic field in the horizontal direction generated by the extracorporeal permanent magnet 25a-1 and the magnetic field in the vertical direction generated by the coil 25a-2. Also, the azimuth angle of the capsule endoscope 10 is controlled by the revolution angle changing action of the extracorporeal permanent magnet 25a-1 by the revolution angle changing unit 25e. Furthermore, the position of the capsule endoscope 10 is controlled by the translating action of the extracorporeal permanent magnet 25a-1 by the plane position changing unit 25b and the vertical position changing unit 25c.

According to modified example 1-5, since an electromagnet can generate a strong magnetic field having uniformity higher compared to a permanent magnet to the guidance region, the tilt angle and the azimuth angle of the capsule endoscope 10 can be more stably controlled. Also, in this case, since the extracorporeal permanent magnet 25a-1 is mainly used only for controlling the position and the azimuth angle of the capsule endoscope 10, the limitation to the shape of the extracorporeal permanent magnet 25a-1 can be alleviated.

(Second Embodiment)

Next, a second embodiment of the present invention will be described.

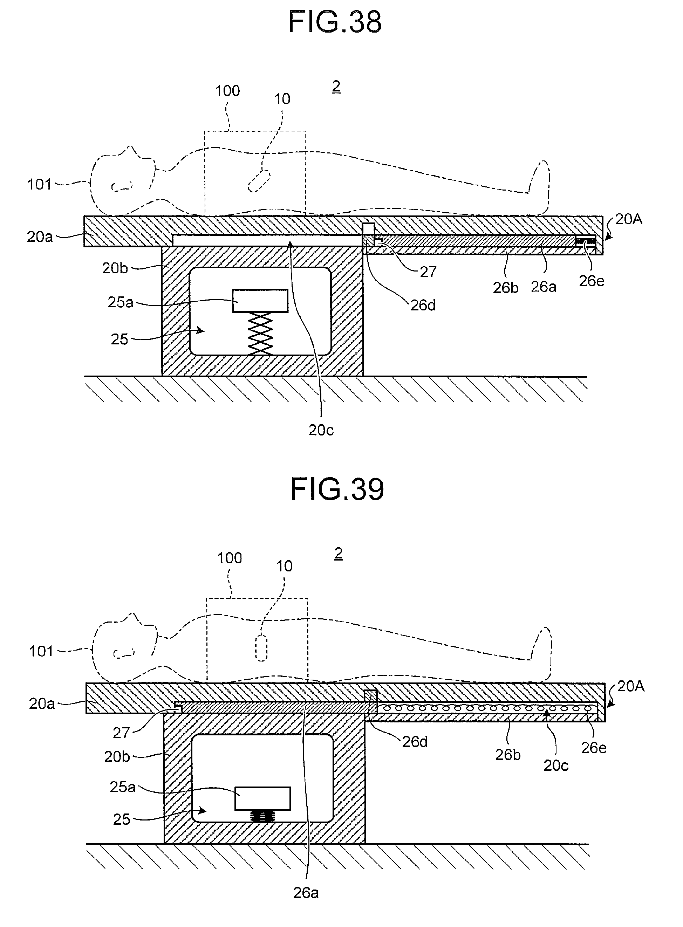

FIG. 38 and FIG. 39 are partial sectional side views schematically illustrating a configuration example of a capsule medical apparatus guidance system according to a second embodiment. As illustrated in FIG. 38 and FIG. 39, a capsule medical apparatus guidance system (hereinafter, merely referred to as a guidance system) 2 according to the second embodiment includes the capsule endoscope 10 and a guidance device 20A. FIG. 38 illustrates a case in which the guidance device 20A is in the magnetic field generating state, and FIG. 39 illustrates a case where the guidance device 20A is in the magnetic field shielding state.

The guidance device 20A has, instead of the drive unit 26c illustrated in FIG. 2, an elastic member 26e that slides the magnetic body member 26a with an elastic force. As the elastic member 26e, there can be used, for example, rubber as well as a spring member such as a wind-up spring, a disk spring and a plate spring. The configuration of each of the other components of the guidance device 20A is similar to that in the first embodiment.

As illustrated in FIG. 38, when the guidance device 20A is in the magnetic field generating state, the elastic member 26e shrinks while pressing the magnetic body member 26a with the elastic force thereof. At this time, the position of the magnetic body member 26a is fixed by the fixing unit 26d.

As illustrated in FIG. 39, when the fixing unit 26d is moved upward to release the fixing state of the magnetic body member 26a, the magnetic body member 26a slides with an elastic force caused by the elastic member 26e and moves to the concave 20c below the effective magnetic field area 100 (above the magnetic field generating unit 25). Accordingly, the guidance device 20A is shifted to the magnetic field shielding state. Here, operation of the fixing unit 26d for releasing the fixing state of the magnetic body member 26a may be performed manually by a user, or the fixing unit 26d may be operated by a drive unit that acts under control of the control unit 28.

According to the second embodiment described above, the magnetic body member 26a is moved by the elastic force of the elastic member 26e. Therefore, for example, even when a situation like sudden loss of electric power occurs, the guidance device 20A can be quickly shifted to the magnetic field shielding state.

Here, when shifting the guidance device 20A from the magnetic field shielding state to the magnetic field generating state, a user may manually move the positions of the magnetic body member 26a and the fixing unit 26d, or the magnetic body member 26a may be moved in the horizontal direction (in the right direction of the diagram) by a separately disposed drive unit that acts under control of the control unit 28.

(Third Embodiment)

Next, a third embodiment of the present invention will be described.

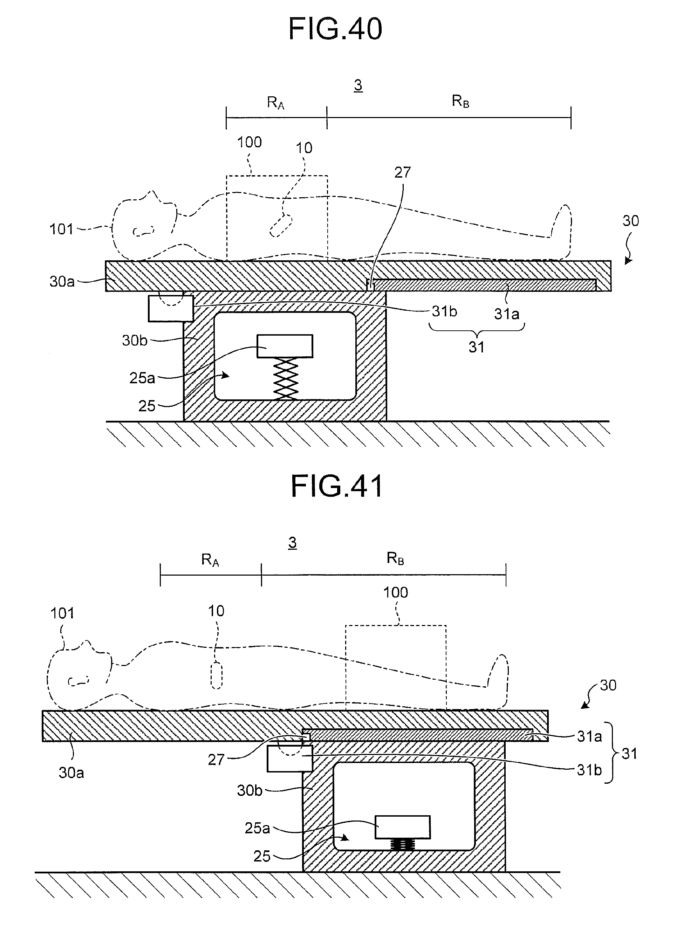

FIG. 40 and FIG. 41 are partial sectional side views schematically illustrating a configuration example of a capsule medical apparatus guidance system according to the third embodiment. As illustrated in FIG. 40 and FIG. 41, a capsule medical apparatus guidance system (hereinafter, merely referred to as a guidance system) 3 according to the third embodiment includes the capsule endoscope 10 and a guidance device 30. FIG. 40 illustrates a case in which the guidance device 30 is in the magnetic field generating state, and FIG. 41 illustrates a case where the guidance device 30 is in the magnetic field shielding state.

The configuration of the guidance system 3 is generally similar to the guidance system 1 illustrated in FIG. 1, and only the configurations of a bed 30a and a shielding unit 31 described later are different from the guidance system 1.

The guidance device 30 includes a bed 30a as a placing table on which the subject 101 is placed. The bed 30a is slidably disposed with respect to a leg 30b that supports the bed 30a. The main surface (the subject placing surface) of the bed 30a contains a region $R_A$ on which the examination target region (for example, the stomach) of the subject 101 is placed, and a region $R_B$ on which the non-examination target region (for example, the leg) is placed. The position of this bed 30a relative to the leg 30b is switched by a drive unit 31b described later between the position of a state where the region $R_A$ is inserted between the magnetic field generating unit 25 and the effective magnetic field area 100 (the magnetic field generating state), and the position of a state where the region $R_A$ is removed from between the magnetic field generating unit 25 and the effective magnetic field area 100 (the magnetic field shielding state).

The magnetic field generating unit 25 for forming a magnetic field to the effective magnetic field area 100 is housed inside the leg 30b. Here, in order to inhibit leakage of the magnetic field generated by the magnetic field generating unit 25 into a space other than the effective magnetic field area 100 (for example, in the side surface direction of the leg 30b), the leg 30b is preferably made of a ferromagnetic substance such as an iron plate.

The guidance device 30 includes a shielding unit 31 that shields the magnetic field generated by the magnetic field generating unit 25 to the effective magnetic field area 100. The shielding unit 31 has a magnetic body member 31a and a drive unit 31b. The magnetic body member 31a is attached to the lower surface of the bed 30a, and the drive unit 31b acts under control of the control unit 28 to allow the bed 30a to move together with the magnetic body member 31a.

The magnetic body member 31a is made of, for example, a ferromagnetic substance such as an iron plate. The material and the size of the magnetic body member 31a are similar to the magnetic body member 26a described in the first embodiment. Such a magnetic body member 31a is fixed in a concave disposed on the lower surface of the bed 30a through adhesion, mechanical fastening, and the like. The position of the magnetic body member 31a relative to the bed 30a is determined so that when shifted to the magnetic field shielding state, the magnetic body member 31a covers at least the region above the magnetic field generating unit 25. In the third embodiment, the magnetic body member 31a is arranged in a portion of the region $R_B$.

The drive unit 31b moves the bed 30a attached with the magnetic body member 31a one-dimensionally (for example, in the height direction of the subject 101) within the horizontal plane, in order to switch the position of the bed 30a between the position in the magnetic field generating state and the position in the magnetic field shielding state.

The guidance device 30, as illustrated in FIG. 40, moves the bed 30a so that the region $R_A$ is located above the magnetic field generating unit 25 during examination. Accordingly, the magnetic body member 31a is removed from between the magnetic field generating unit 25 and the effective magnetic field area 100, and the magnetic field generated in the effective magnetic field area 100 by the magnetic field generating unit 25 leads to a state (a magnetism generating state) that allows the magnetic guidance of the capsule endoscope 10. On the other hand, the guidance device 30, as illustrated in FIG. 41, moves the bed 30a such that the region $R_A$ departs from above the magnetic field generating unit 25 before and after examination or during an emergency stop. This leads to the magnetic field shielding state where the magnetic body member 31a is inserted between the magnetic field generating unit 25 and the effective magnetic field area 100.

Thus, in the third embodiment, since the shielding of a magnetic field by the magnetic body member 31a and the movement of the bed 30a are performed in conjunction with each other, the effect of a magnetic field on the capsule endoscope 10 in the magnetic field shielding state can be further reduced.

Here, in the third embodiment, the bed 30a may also be moved due to an elastic member such as a spring in a similar manner to the second embodiment, instead of the drive unit 31b.

(Fourth embodiment)

Next, a fourth embodiment of the present invention will be described.

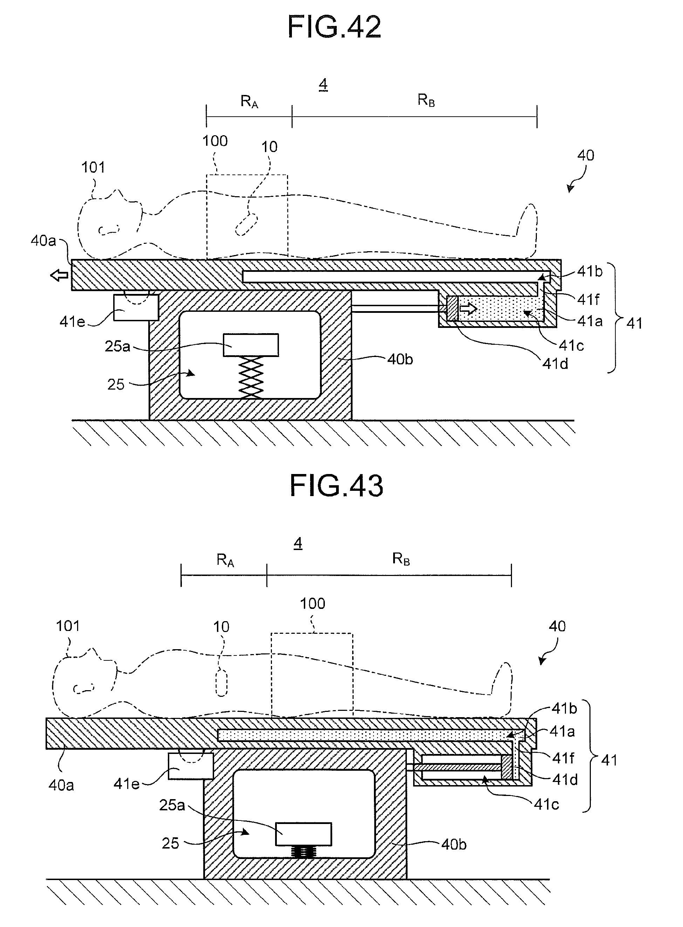

FIG. 42 and FIG. 43 are partial sectional side views schematically illustrating a configuration example of the capsule medical apparatus guidance system according to the fourth embodiment. As illustrated in FIG. 42 and FIG. 43, a capsule medical apparatus guidance system (hereinafter, merely referred to as a guidance system) 4 according to the fourth embodiment includes the capsule endoscope 10 and a guidance device 40. FIG. 42 illustrates a case in which the guidance device 40 is in the magnetic field generating state, and FIG. 43 illustrates a case where the guidance device 40 is in the magnetic field shielding state.

The configuration of the guidance system 4 is generally similar to the guidance system 1 illustrated in FIG. 1, and only the configurations of a bed 40a and a shielding unit 41 described later are different from the guidance system 1.

The guidance device 40 includes a bed 40a as a placing table on which the subject 101 is placed. The bed 40a is slidably disposed with respect to a leg 40b that supports the bed 40a. The main surface of the bed 40a contains a region $R_A$ on which the examination target region of the subject 101 is placed, and a region $R_B$ on which the non-examination target region is placed. The position of this bed 40a relative to the leg 40b is switched by a drive unit 41e described later between the position of a state where the region $R_A$ is inserted between the magnetic field generating unit 25 and the effective magnetic field area 100 (the magnetic field generating state), and the position of a state where the region $R_A$ is removed from between the magnetic field generating unit 25 and the effective magnetic field area 100 (the magnetic field shielding state).

The magnetic field generating unit 25 for forming a magnetic field to the effective magnetic field area 100 is housed inside the leg 40b. Here, in order to inhibit the leakage of the magnetic field generated by the magnetic field generating unit 25 into a space other than the effective magnetic field area 100 (for example, in the side surface direction of the leg 40b), the leg 40b may be made of a ferromagnetic substance such as an iron plate.

A guidance device 40 includes a shielding unit 41 that shields the magnetic field generated by the magnetic field generating unit 25 to the effective magnetic field area 100. The shielding unit 41 has a magnetic fluid 41a, a magnetic fluid housing unit 41b disposed inside the bed 40a, a magnetic fluid storage unit 41c disposed below the bed 40a, a piston 41d that moves the magnetic fluid 41a into the magnetic fluid housing unit 41b through a linking hole 41f, and a drive unit 41e that acts under control of the control unit 28 to move the bed 40a.

The magnetic fluid 41a is a fluid having magnetic properties. For example, a dispersion of magnetic body particles such as magnetite in liquid such as water or oil is used. Such a magnetic fluid 41a is stored in the magnetic fluid storage unit 41c when the guidance device 40 is in the magnetic field generating state, and is housed in the magnetic fluid housing unit 41b when the guidance device 40 is in the magnetic field shielding state.

The region where the magnetic fluid housing unit 41b is disposed is determined so that when shifted to the magnetic field shielding state, the magnetic fluid housing unit 41b covers at least the region above the magnetic field generating unit 25. In the fourth embodiment, the magnetic fluid housing unit 41b is disposed in the region $R_B$ and a portion of the region $R_A$.

The magnetic fluid storage unit 41c has a volume substantially equal to the magnetic fluid housing unit 41b, and disposed, for example, in the end region of the bed 40a.

The magnetic fluid housing unit 41b and the magnetic fluid storage unit 41c communicate with each other through the linking hole 41f.

The piston 41d is a magnetic fluid moving means that is disposed within the magnetic fluid storage unit 41c and that works in conjunction with movement of the bed 40a. The piston 41d is moved in the right direction of the diagram to the magnetic fluid storage unit 41c, thereby to extrude the magnetic fluid 41a in the magnetic fluid storage unit 41c into the magnetic fluid housing unit 41b through the linking hole 41f. Also, the piston 41d is moved in the left direction of the diagram to the magnetic fluid storage unit 41c, thereby to suck the magnetic fluid 41a in the magnetic fluid housing unit 41b into the magnetic fluid storage unit 41c through the linking hole 41f.

The drive unit 41e moves the bed 40a one-dimensionally (for example, in the height direction of the subject 101) within the horizontal plane, so that the position of the bed 40a is switched between the position in the magnetic field generating state and the position in the magnetic field shielding state. The relative position of the piston 41d to the magnetic fluid storage unit 41c is changed in conjunction with the shifting of the position of the bed 40a.

The guidance device 40, as illustrated in FIG. 42, moves the bed 40a so that the region $R_A$ is located above the magnetic field generating unit 25 during examination. Accordingly, the magnetic fluid 41a is sucked into the magnetic fluid storage unit 41c so that the magnetic fluid housing unit 41b becomes empty, and the magnetic field generated in the effective magnetic field area 100 by the magnetic field generating unit 25 leads to a state (the magnetic field generating state) that allows the magnetic guidance of the capsule endoscope 10. On the other hand, the guidance device 40, as illustrated in FIG. 43, moves the bed 40a so that the region $R_A$ departs from above the magnetic field generating unit 25 before and after examination or during an emergency stop. Accordingly, the magnetic fluid 41a in the magnetic fluid storage unit 41c is extruded into the magnetic fluid housing unit 41b, and the magnetic fluid housing unit 41b filled with the magnetic fluid 41a is inserted between the magnetic field generating unit 25 and the effective magnetic field area 100. That is, the magnetic field shielding state is led.

As described above, in the fourth embodiment, by positioning the examination target region of the subject 101 off the effective magnetic field area 100 while inserting the magnetic fluid 41a between the magnetic field generating unit 25 and the effective magnetic field area 100, the magnetic field shielding state is achieved.

Therefore, the effect of a magnetic field on the capsule endoscope 10 in the magnetic field shielding state can be further reduced.

In the fourth embodiment, the piston 41d moves in conjunction with the movement of the bed 40a. However, the bed 40a may be fixed to the leg 40b to allow only the piston 41d to be moved, so that the magnetic fluid 41a circulates between the magnetic fluid housing unit 41b and the magnetic fluid storage unit 41c. In this case, the magnetic fluid housing unit 41b may be disposed in the region of the bed 40a between the magnetic field generating unit 25 and the effective magnetic field area 100 (that is, a region containing the region $R_A$).

(Fifth Embodiment)

Next, a fifth embodiment of the present invention will be described.

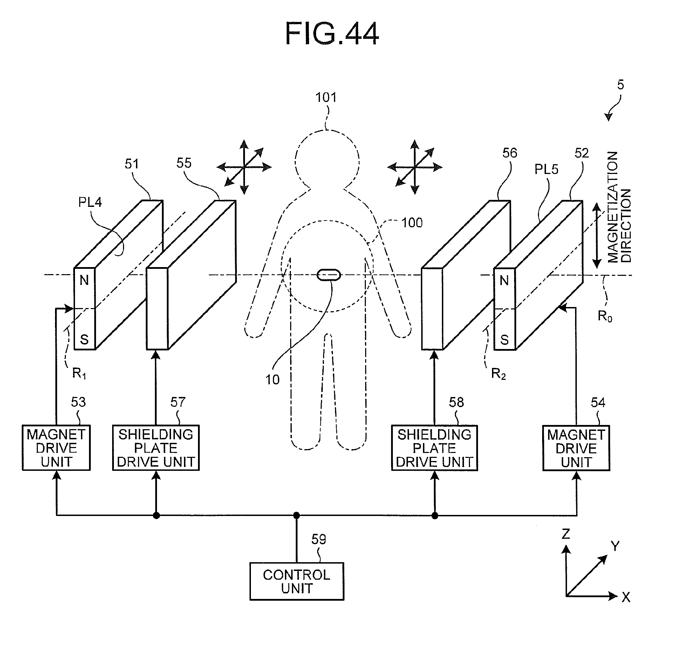

FIG. 44 is a perspective view schematically illustrating a configuration example of a capsule medical apparatus guidance system according to the fifth embodiment. As illustrated in FIG. 44, a capsule medical apparatus guidance system (hereinafter, also merely referred to as a guidance system) 5 according to the fifth embodiment includes: the capsule endoscope 10 that contains the permanent magnet 19 therein and that is introduced into the subject 101; permanent magnets 51 and 52 that are faced to each other on both sides of the subject 101; magnet drive units 53 and 54 that drive the permanent magnets 51 and 52 respectively; shielding plates 55 and 56 disposed in an insertable and removable manner between the permanent magnets 51 and 52 and the subject 101; shielding plate drive units 57 and 58 that drive the shielding plates 55 and 56 respectively; and a control unit 59 that controls actions of the magnet drive units 53 and 54 and the shielding plate drive units 57 and 58. The capsule endoscope 10 is confined by a magnetic field generated within the subject 101 by the permanent magnets 51 and 52. The position, the tilt angle and the azimuth angle of the capsule endoscope 10 are changed in accordance with the position, elevation angle θ and revolution angle ψ of the permanent magnets 51 and 52 driven by the magnet drive units 53 and 54.

The permanent magnets 51 and 52 are permanent magnets of the same type as and having the same rectangular parallelepiped shape as each other. The permanent magnets 51 and 52 each have a magnetization direction parallel to four planes of the rectangular parallelepiped. One plane (hereinafter, referred to as capsule facing planes PL4 and PL5) among these is directed toward the subject 101. The magnetization directions of the capsule facing planes PL4 and PL5 are placed in parallel so as to be mirror-symmetrical to each other. Here, the magnetization directions of the permanent magnets 51 and 52 are directed toward the vertical direction (z-axis direction) when the guidance of the capsule endoscope 10 is not performed. Hereinafter, among the directions orthogonal to the vertical direction when the guidance of the capsule endoscope 10 is not performed, the direction orthogonal to the capsule facing planes PL4 and PL5 is defined as an x-axis direction, and the direction parallel to the capsule facing planes PL4 and PL5 is defined as a y-axis direction.

Each of the permanent magnets 51 and 52 has a shape in which, among the lengths of the sides in three directions of the rectangular parallelepiped shape, the length of a side in a direction orthogonal to the capsule facing planes PL4 and PL5 (in the x-axis direction in FIG. 44) is shorter than the length of a side in one direction contained in the capsule facing planes PL4 and PL5 (a magnetization direction or a direction orthogonal to the magnetization direction, in the z-axis direction or the y-axis direction in FIG. 44). Preferably, the permanent magnets 51 and 52 each have a flat plate shape in which, among the lengths of the sides in three directions of the rectangular parallelepiped shape, the length in a direction orthogonal to the capsule facing planes PL4 and PL5 is the shortest.

The permanent magnets 51 and 52 are configured in such a manner as being capable of translating together in the horizontal direction and the vertical direction. Accordingly, the position of the capsule endoscope 10 within the subject 101 can be controlled. Further, the translation of the permanent magnets 51 and 52 within the vertical plane changes the position of the capsule endoscope 10 within the vertical plane. For example, the translation of the permanent magnets 51 and 52 within the horizontal plane changes the position of the capsule endoscope 10 within the horizontal plane.

The permanent magnets 51 and 52 are configured in such a manner as being capable of rotating around an axis $R_0$ that is orthogonal to the capsule facing planes PL4 and PL5 and extending through respective centers, and around axes $R_1$ and $R_2$ that are orthogonal to the magnetization direction within the capsule facing planes PL4 and PL5. Accordingly, the azimuth angle and the tilt angle of the capsule endoscope 10 within the subject 101 can be controlled. For example, the rotation (revolution) of the permanent magnets 51 and 52 around the axis $R_0$ while maintaining the positional relationship between the permanent magnets 51 and 52 is followed by the capsule endoscope 10 to change the azimuth angle. Also, the tilting of the permanent magnets 51 and 52 relative to the axes $R_1$ and $R_2$ while maintaining the positional relationship between the permanent magnets 51 and 52 is followed by the capsule endoscope 10 to be tilted.

Furthermore, the permanent magnets 51 and 52 are configured in such a manner as being capable of changing the distance between the permanent magnets 51 and 52. The change of the distance between the permanent magnets 51 and 52 can cause the magnetic field strength in the effective magnetic field area 100 to be changed. In a guidance system 5, the position of the permanent magnets 51 and 52 when the distance between the permanent magnets 51 and 52 is the largest within the installable range (that is, when the magnetic field strength in the effective magnetic field area 100 is the lowest) is set as an initial position.

The shielding plates 55 and 56 are each a member made of a ferromagnetic substance such as an iron plate. The shielding plates 55 and 56 may have a material and a size (width x length) that allow the magnetic field generated by the permanent magnets 51 and 52 to be shielded in the effective magnetic field area 100. In the present fifth embodiment, the areas of the main surfaces of the permanent magnets 51 and 52 as well as the shielding plates 55 and 56 are approximately the same.

The shielding plate drive units 57 and 58 drive the shielding plates 55 and 56 in the vertical direction, thereby to insert and remove the shielding plates 55 and 56 into and from between the effective magnetic field area 100 and the permanent magnets 51 and 52. When the shielding plates 55 and 56 are inserted between the effective magnetic field area 100 and the permanent magnets 51 and 52, the guidance system 5 becomes in the magnetic field shielding state, and when the shielding plates 55 and 56 are removed from between the effective magnetic field area 100 and the permanent magnets 51 and 52, the guidance system 5 becomes in the magnetic field generating state.

According to such a fifth embodiment, the subject 101 can be examined in a standing posture, and furthermore, the magnetic field generating state and the magnetic field shielding state in the guidance system 5 can be switched.

Here, like the fifth embodiment, when a permanent magnet is disposed laterally to the subject 101, a capsule endoscope guidance system that examines a subject in a sitting posture can also be configured. In this case, a permanent magnet and a shielding plate may be disposed on a backrest or an armrest of a chair on which a subject sits and which is provided as a placing table.

The embodiments described above are only examples for implementing the present invention, and the present invention is not limited to these. Also, the present invention can generate various inventions by appropriately combining a plurality of components disclosed in the embodiments and modified examples. The present invention can be variously modified depending on specifications and the like. Furthermore, it is obvious from the above description that other various embodiments are possible within the scope of the present invention.

(Note 1)

A guidance device for applying a magnetic field to a capsule medical apparatus within which a first permanent magnet is arranged when the capsule medical apparatus is introduced into a subject, to guide the capsule medical apparatus within the subject, the guidance device including:

a second permanent magnet configured to be disposed outside the subject, the second permanent magnet having a first plane containing a magnetization direction and a first direction orthogonal to the magnetization direction, and being configured to confine the capsule medical apparatus within a region facing the first plane; and

a shielding unit configured to shield a magnetic field generated by the second permanent magnet in an effective magnetic field area where the magnetic field capable of guiding the capsule medical apparatus is generated by the second permanent magnet, and configured to switch between a first state in which the magnetic field is not shielded in the effective magnetic field area and a second state in which the magnetic field is shielded in the effective magnetic field area.

(Note 2)

The guidance device according to Note 1, wherein the shielding unit includes:

a magnetic body; and

a drive unit configured to insert and remove the magnetic body into and from between the second permanent magnet and the effective magnetic field area.

(Note 3)

The guidance device according to Note 2, wherein the magnetic body has a plate shape.

(Note 4)

The guidance device according to Note 2, wherein the drive unit is an elastic member for pressing the magnetic body with an elastic force.

(Note 5)

The guidance device according to any one of Notes 1 to 4, further including:

a detecting unit configured to detect a shielding state of the magnetic field by the shielding unit; and

a notifying unit configured to notify a detection result by the detecting unit.

(Note 6)

The guidance device according to Note 5, wherein the notifying unit is configured to notify the detection result using visual information or auditory information.

(Note 7)

A capsule medical apparatus guidance system including:

the guidance device according to any one of Notes 1 to 6; and
the capsule medical apparatus in which the first permanent magnet is arranged.

(Note 8)

A capsule medical apparatus guidance system including:

a guidance device for applying a magnetic field to a capsule medical apparatus within which a first permanent magnet is arranged when the capsule medical apparatus is introduced into a subject, to guide the capsule medical apparatus within the subject; and
the capsule medical apparatus in which the first permanent magnet is arranged,
wherein the guidance device includes a second permanent magnet configured to be disposed outside the subject, the second permanent magnet having a first plane containing a magnetization direction and a first direction orthogonal to the magnetization direction, and being configured to confine the capsule medical apparatus within a region facing the first plane,
wherein the second permanent magnet has a length in the first direction longer than a length in the magnetization direction.

(Note 9)

The capsule medical apparatus guidance system according to Note 7 or 8,
wherein the capsule medical apparatus is guided by the guidance device in a liquid introduced into the subject, and
a gravity center of the capsule medical apparatus is arranged away from a geometric center of the capsule medical apparatus in a direction different from a magnetization direction of the first permanent magnet.

Reference Signs List

| | |
|---|---|
| 1 to 5 | CAPSULE MEDICAL APPARATUS GUIDANCE SYSTEM |
| 10 | CAPSULE ENDOSCOPE |
| 11A, 11B | IMAGING UNIT |
| 12 | CAPSULE-SHAPED CASING |
| 12a | TUBULAR CASING |
| 12b, 12c | DOME-SHAPED CASING |
| 13A, 13B | ILLUMINATION UNIT |
| 14A, 14B | OPTICAL SYSTEM |
| 15A, 15B | IMAGING ELEMENT |
| 16 | WIRELESS COMMUNICATION UNIT |
| 16a | ANTENNA |
| 17 | CONTROL UNIT |
| 18 | POWER SOURCE UNIT |
| 19 | PERMANENT MAGNET |
| 20, 20A, 30, 40 | GUIDANCE DEVICE |
| 20a, 30a, 40a | BED |
| 20b, 30b, 40b | LEG |
| 20c | CONCAVE |
| 21 | RECEIVER |
| 21a | ANTENNA |
| 22 | POSITION DETECTOR |
| 23a | DISPLAY UNIT |
| 23b | NOTIFYING UNIT |
| 24 | OPERATION INPUT UNIT |
| 25 | MAGNETIC FIELD GENERATING UNIT |
| 25a, 25a-1 | EXTRACORPOREAL PERMANENT MAGNET |
| 25a-2 | COIL |
| 25b | PLANE POSITION CHANGING UNIT |
| 25c | VERTICAL POSITION CHANGING UNIT |
| 25d | ELEVATION ANGLE CHANGING UNIT |
| 25e | REVOLUTION ANGLE CHANGING UNIT |
| 26, 31, 41 | SHIELDING UNIT |
| 26a, 31a | MAGNETIC BODY MEMBER |
| 26b | SUPPORT |
| 26c, 31b, 41e | DRIVE UNIT |
| 26d | FIXING UNIT |
| 26e | ELASTIC MEMBER |
| 27 | SHIELDING STATE DETECTOR |
| 28 | CONTROL UNIT |
| 29 | STORAGE UNIT |
| 41a | MAGNETIC FLUID |
| 41b | MAGNETIC FLUID HOUSING UNIT |
| 41c | MAGNETIC FLUID STORAGE UNIT |
| 41d | PISTON |
| 41f | LINKING HOLE |
| 51, 52 | PERMANENT MAGNET |
| 53, 54 | MAGNET DRIVE UNIT |
| 55, 56 | SHIELDING PLATE |
| 57, 58 | SHIELDING PLATE DRIVE UNIT |
| 59 | CONTROL UNIT |
| 61, 62 | JOY STICK |
| 64U | UP BUTTON |
| 64B | DOWN BUTTON |
| 65 | CAPTURE BUTTON |
| 66 | APPROACH BUTTON |
| 100 | EFFECTIVE MAGNETIC FIELD AREA |

101 SUBJECT

## Claims

1. A guidance device for applying a magnetic field to a capsule medical apparatus within which a first permanent magnet is arranged when the capsule medical apparatus is introduced into a subject, to guide the capsule medical apparatus within the subject, the guidance device comprising

   a second permanent magnet configured to be disposed outside the subject, the second permanent magnet having a first plane containing a magnetization direction and a first direction orthogonal to the magnetization direction, and being configured to confine the capsule medical apparatus within a region facing the first plane,

   wherein the second permanent magnet has a length in the first direction longer than a length in the magnetization direction.

2. The guidance device according to claim 1, wherein the length in the magnetization direction is 2/3 or less of the length in the first direction.

3. The guidance device according to claim 1, wherein a length in a second direction orthogonal to the magnetization direction and the first direction in the second permanent magnet is shorter than the length in the first direction.

4. The guidance device according to claim 1, wherein a value K given by

$$K = \sqrt{\frac{L_y^2}{L_x \times L_z}}$$

   is more than 1 and 22.6 or less, where $L_x$ is the length in the magnetization direction, $L_y$ is the length in the first direction, and $L_z$ is a length in a second direction orthogonal to the magnetization direction and the first direction.

5. The guidance device according to claim 4, wherein the length in the magnetization direction is the length in the second direction or longer.

6. The guidance device according to claim 1, further comprising a revolution angle changing unit configured to rotate the second permanent magnet around an axis orthogonal to an optional reference plane parallel to the first direction.

7. The guidance device according to claim 1, further comprising a translation mechanism configured to translate the second permanent magnet within an optional reference plane parallel to the first direction.

8. The guidance device according to claim 1, further comprising an elevation angle changing unit configured to rotate the second permanent magnet around an axis parallel to the first direction, to change an elevation angle of the magnetization direction with respect to an optional reference plane parallel to the first direction.

9. The guidance device according to any one of claims 6 to 8, wherein the reference plane is parallel to a horizontal plane.

10. The guidance device according to claim 1, further comprising a shielding unit configured to shield a magnetic field generated by the second permanent magnet in an effective magnetic field area as an area where the magnetic field capable of guiding the capsule medical apparatus is generated by the second permanent magnet, and configured to switch between a first state in which the magnetic field is not shielded in the effective magnetic field area and a second state in which the magnetic field is shielded in the effective magnetic field area.

11. The guidance device according to claim 10, wherein the shielding unit comprises:

    a magnetic body; and
    a drive unit configured to insert and remove the magnetic body into and from between the second permanent magnet and the effective magnetic field area.

12. The guidance device according to claim 11, further comprising

    a placing table on which the subject is configured to be placed, the placing table having a first region on which an examination target region of the subject is configured to be placed and a second region on which a non-examination target region of the subject is configured to be placed,

    wherein the magnetic body is arranged within the second region on the placing table, and

    the drive unit is configured to drive the magnetic body via the placing table.

13. The guidance device according to claim 10, wherein the shielding unit comprises:

    a magnetic fluid;
    a magnetic fluid housing unit that is disposed between the second permanent magnet and the effective magnetic field area and is configured to house the magnetic fluid;

a magnetic fluid storage unit that communicates with the magnetic fluid housing unit; and
a magnetic fluid moving unit configured to move the magnetic fluid between the magnetic fluid housing unit and the magnetic fluid storage unit.

14. The guidance device according to claim 10, further comprising:

a placing table on which the subject is configured to be placed, the placing table having a first region on which an examination target region of the subject is configured to be placed and a second region on which a non-examination target region of the subject is configured to be placed; and
a drive unit configured to drive the placing table to switch a position of the placing table between a first position where the first region is inserted between the second permanent magnet and the effective magnetic field area and a second position where the first region is removed from between the second permanent magnet and the effective magnetic field area,
wherein the shielding unit comprises:

a magnetic fluid;
a magnetic fluid housing unit that is disposed in a region which is inserted between the second permanent magnet and the effective magnetic field area when the placing table is in the second position, and is configured to house the magnetic fluid;
a magnetic fluid storage unit that communicates with the magnetic fluid housing unit; and
a magnetic fluid moving unit configured to work in conjunction with the drive unit and configured to move the magnetic fluid between the magnetic fluid housing unit and the magnetic fluid storage unit,
wherein the magnetic fluid moving unit moves the magnetic fluid to the magnetic fluid housing unit when the placing table is shifted from the first position to the second position.

15. The guidance device according to claim 13 or 14, wherein the magnetic fluid moving unit is a piston that is disposed in the magnetic fluid storage unit for changing a volume within the magnetic fluid storage unit to inject the magnetic fluid from the magnetic fluid storage unit into the magnetic fluid housing unit or for sucking the magnetic fluid from the magnetic fluid housing unit into the magnetic fluid storage unit.

16. The guidance device according to claim 10, further comprising:

a detecting unit configured to detect a shielding state of the magnetic field by the shielding unit; and
a control unit configured to control switching between the first state and the second state depending on a detection result by the detecting unit and a status of an examination by the capsule medical apparatus.

17. The guidance device according to claim 10, further comprising:

a detecting unit configured to detect a shielding state of the magnetic field by the shielding unit; and
a notifying unit configured to notify a detection result by the detecting unit.

# FIG.1

GUIDANCE DEVICE

MAGNETIC FIELD GENERATING UNIT ⌐25

EXTRACORPOREAL PERMANENT MAGNET ⌐25a

| PLANE POSITION CHANGING UNIT ⌐25b | VERTICAL POSITION CHANGING UNIT ⌐25c | ELEVATION ANGLE CHANGING UNIT ⌐25d | REVOLU-TION ANGLE CHANGING UNIT ⌐25e |

CONTROL UNIT ⌐28

SHIELDING UNIT ⌐26

SHIELDING STATE DETECTOR ⌐27

STORAGE UNIT ⌐29

RECEIV-ER ⌐21

POSITION DETEC-TOR ⌐22

DISPLAY UNIT ⌐23a

NOTIFY-ING UNIT ⌐23b

OPERA-TION INPUT UNIT ⌐24

# FIG.2

# FIG.3

# FIG.4

REVO-
LUTION          PL1

MAGNETI-                    VERTICAL
ZATION                     DIRECTION
DIRECTION

25a

Y$_c$    ROTA-
         TION

HORIZONTAL
PLANE

~10

Z
Y
X

# FIG.5

PERMANENT
MAGNET
19                          10

14B  13B  11B  16a  16        18   17  11A  13A  14A

WIRELESS COMMUNICATION UNIT

POWER SOURCE UNIT

CONTROL UNIT

La

15B

13B                              13A

15A

12c   12a   12b

12

# FIG.6

# FIG.7

# FIG.8

# FIG.9

# FIG.10

(a)

MAGNETIC FIELD
STRENGTH

HORIZONTAL
POSITION

(b)

10

19

PL1

25a

# FIG.11

(a)

VERTICAL
POSITION

MAGNETIC FIELD
STRENGTH

(b)

19

10

PL1

25a

# FIG.12

(a)

(b)

# FIG.13

# FIG.14

Patient ID: 123456    Patient Name

Sex
Age
D.O.Birth
Exam.Data
00:01.53

00:08:37  00:08:38  00:09:39  00:09:42  00:08:39  00:08:39  00:08:40  00:08:40

FIG.15

START

CHECK MAGNETIC FIELD SHIELDING STATE —S101

CHECK POSITION OF EXTRACORPOREAL PERMANENT MAGNET —S102

RECOGNIZE PLACEMENT OF SUBJECT —S103

TURN CAPSULE POWER SOURCE ON, AND CHECK ACQUISITION OF IMAGE —S104

RECOGNIZE SWALLOWING OF CAPSULE —S105

SHIFT INTO MAGNETIC FIELD GENERATING STATE (WEAK MAGNETIC FIELD GENERATING STATE) —S106

RECEIVE BODY POSITION INFORMATION —S107

START GUIDANCE OF CAPSULE (SHIFT INTO NORMAL MAGNETIC FIELD GENERATING STATE) —S108

ACQUIRE AND DISPLAY IMAGE —S109

S110
EMER-GENCY STOP TRIGGER ON?

YES

S111
NOTIFICATION AND EMERGENCY SHIELDING

NO

S112
HAS ACQUISITION OF ALL IMAGES BEEN COMPLETED?

NO

YES

S113
STOP GUIDANCE OF CAPSULE (SHIFT INTO WEAK MAGNETIC FIELD GENERATING STATE)

S114
IS BODY POSITION INFORMATION INPUT?

NO

YES S115
SHIFT INTO WEAK MAGNETIC FIELD GENERATING STATE

S116
SHIFT INTO MAGNETIC FIELD SHIELDING STATE

STOP

# FIG.16

# FIG.17

MAGNETIC FIELD STRENGTH → y-AXIS GRADIENT

x-AXIS GRADIENT

SIMULATION POSITION

z-AXIS GRADIENT

PL2

PERMANENT MAGNET

$L_z$

$L_y$

$L_x$

MAGNETIZATION DIRECTION

# FIG.18

| TYPE | xyz | x-y-z | x-z-y | y-x-z | y-z-x | z-x-y | z-y-x |
|---|---|---|---|---|---|---|---|
| LENGTH $L_x$ IN x-AXIS DIRECTION | 100 | 200 | 200 | 100 | 50 | 100 | 50 |
| LENGTH $L_y$ IN y-AXIS DIRECTION | 100 | 100 | 50 | 200 | 200 | 50 | 100 |
| LENGTH $L_z$ IN z-AXIS DIRECTION | 100 | 50 | 100 | 50 | 100 | 200 | 200 |

# FIG.19

MAGNETIC FIELD
STRENGTH

# FIG.20

MAGNETIC FIELD
GRADIENT
(z-AXIS DIRECTION)

# FIG.21

MAGNETIC FIELD
GRADIENT
(x-AXIS DIRECTION)

DISTANCE FROM CENTRAL AXIS

# FIG.22

MAGNETIC FIELD
GRADIENT
(y-AXIS DIRECTION)

DISTANCE FROM CENTRAL AXIS

# FIG.23

| TYPE | xyz | y-x-z(75) | y-x-z(50) | y-x-z(33) |
|---|---|---|---|---|
| LENGTH $L_X$ IN x-AXIS DIRECTION | 100 | 100 | 100 | 100 |
| LENGTH $L_Y$ IN y-AXIS DIRECTION | 100 | 133 | 200 | 300 |
| LENGTH $L_Z$ IN z-AXIS DIRECTION | 100 | 75 | 50 | 33 |

# FIG.24

MAGNETIC FIELD
STRENGTH

# FIG.25

MAGNETIC FIELD
GRADIENT
(z-AXIS DIRECTION)

# FIG.26

MAGNETIC FIELD
GRADIENT
(x-AXIS DIRECTION)

DISTANCE FROM CENTRAL AXIS

# FIG.27

MAGNETIC FIELD
GRADIENT
(y-AXIS DIRECTION)

DISTANCE FROM CENTRAL AXIS

# FIG.28

RATIO IN
MAGNETIC FIELD
STRENGTH

$L_y/L_z$

# FIG.29

| TYPE | $L_x=100$ | | | $L_x=50$ | | | $L_x=25$ | | |
|---|---|---|---|---|---|---|---|---|---|
| | A1 | A2 | A3 | B1 | B2 | B3 | C1 | C2 | C3 |
| LENGTH $L_X$ IN x-AXIS DIRECTION | 100 | 100 | 100 | 50 | 50 | 50 | 25 | 25 | 25 |
| LENGTH $L_Y$ IN y-AXIS DIRECTION | 100 | 200 | 400 | 200 | 400 | 800 | 400 | 800 | 1600 |
| LENGTH $L_Z$ IN z-AXIS DIRECTION | 100 | 50 | 25 | 100 | 50 | 25 | 100 | 50 | 25 |
| K | 1.0 | 2.8 | 8.0 | 2.8 | 8.0 | 22.6 | 8.0 | 64.0 | 22.6 |

# FIG.30

MAGNETIC FIELD STRENGTH

$L_z$

# FIG.31

# FIG.32

# FIG.33

# FIG.34

| TYPE | x=100 | | | x=50 | | | x=25 | | |
|---|---|---|---|---|---|---|---|---|---|
| | A1 | A2 | A3 | B1 | B2 | B3 | C1 | C2 | C3 |
| MAGNETIC FIELD STRENGTH | ○ | ◎ | ◎ | ○ | ◎ | ◎ | ○ | ○ | △ |
| MAGNETIC FIELD GRADIENT IN z-AXIS | ○ | ◎ | ◎ | ○ | ◎ | ○ | ○ | ○ | △ |
| MAGNETIC FIELD GRADIENT IN x-AXIS | △ | △ | ○ | △ | ○ | ◎ | ○ | ◎ | ◎ |
| MAGNETIC FIELD GRADIENT IN y-AXIS | ◎ | ◎ | ◎ | ◎ | ◎ | △ | ○ | △ | △ |

# FIG.35

(a)

(b)

# FIG.36

# FIG.37

REVOLUTION

25a-2

Zμ

MAGNETI-
ZATION
DIRECTION

PL3

VERTICAL
DIREC-
TION

25

25a-1

HORIZONTAL
PLANE

Z

Y

X

# FIG.38

# FIG.39

# FIG.40

# FIG.41

# FIG.42

# FIG.43

# FIG.44

## INTERNATIONAL SEARCH REPORT

| International application No. |
| --- |
| PCT/JP2013/062851 |

A. CLASSIFICATION OF SUBJECT MATTER
*A61B1/00*(2006.01)i, *A61B5/07*(2006.01)i

According to International Patent Classification (IPC) or to both national classification and IPC

B. FIELDS SEARCHED

Minimum documentation searched (classification system followed by classification symbols)
A61B1/00, A61B5/07

Documentation searched other than minimum documentation to the extent that such documents are included in the fields searched
Jitsuyo Shinan Koho          1922-1996    Jitsuyo Shinan Toroku Koho    1996-2013
Kokai Jitsuyo Shinan Koho    1971-2013    Toroku Jitsuyo Shinan Koho    1994-2013

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| Y<br>A | JP 2007-215583 A  (Hitachi Metals, Ltd.),<br>30 August 2007 (30.08.2007),<br>claim 8; paragraphs [0002], [0048], [0063],<br>[0068], [0070]; fig. 1, 14, 15<br>(Family: none) | 1-11,13,16,<br>17<br>12,14,15 |
| Y<br>A | JP 2011-147785 A  (Novineon Healthcare<br>Technology Partners GmbH),<br>04 August 2011 (04.08.2011),<br>paragraphs [0023], [0033]; fig. 5<br>& US 2011/0184235 A1    & EP 2347699 A1<br>& BRA PI1100715 | 1-11,13,16,<br>17<br>12,14,15 |

| ☒ | Further documents are listed in the continuation of Box C. | ☐ | See patent family annex. |
| --- | --- | --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>    05 August, 2013 (05.08.13) | Date of mailing of the international search report<br>    13 August, 2013 (13.08.13) |
| --- | --- |
| Name and mailing address of the ISA/<br>    Japanese Patent Office | Authorized officer |
| Facsimile No. | Telephone No. |

Form PCT/ISA/210 (second sheet) (July 2009)

**INTERNATIONAL SEARCH REPORT**

| | International application No. |
|---|---|
| | PCT/JP2013/062851 |

C (Continuation).   DOCUMENTS CONSIDERED TO BE RELEVANT

| Category* | Citation of document, with indication, where appropriate, of the relevant passages | Relevant to claim No. |
|---|---|---|
| Y | WO 2007/37380 A1 (Hitachi Metals, Ltd.), 05 April 2007 (05.04.2007), paragraph [0059] & JP 4893626 B & US 2009/0231073 A1 & EP 1929943 A1 & IL 190410 A | 7,9 |
| Y | JP 2012-71176 A (Olympus Medical Systems Corp.), 12 April 2012 (12.04.2012), paragraph [0477]; fig. 76 & US 2007/0221233 A1 & EP 1969989 A1 & WO 2007/077922 A1 & KR 10-2008-0075536 A & CN 101351146 A | 8,9 |
| Y A | WO 2007/83708 A1 (Olympus Medical Systems Corp.), 26 July 2007 (26.07.2007), paragraphs [0077] to [0080]; fig. 19 to 22 & JP 2012-152607 A & US 2007/0191671 A1 & EP 1982635 A1 & CN 101374450 A & KR 10-2008-0088635 A | 10,11,13,16, 17 12 |
| Y A | JP 2007-167265 A (President of National Cancer Center), 05 July 2007 (05.07.2007), claims 1, 2; paragraphs [0013] to [0021]; fig. 1 (Family: none) | 10,11,13,16, 17 14,15 |
| Y | WO 2011/118253 A1 (Olympus Medical Systems Corp.), 29 September 2011 (29.09.2011), paragraph [0056] & JP 4932971 B & US 2012/0095290 A1 & EP 2481337 A1 & CN 102665530 A | 16 |
| Y | WO 2011/87037 A1 (Olympus Medical Systems Corp.), 21 July 2011 (21.07.2011), claims 1, 25, 26; paragraphs [0071], [0267], [0279], [0287] & JP 4875788 B & US 2012/0010480 A1 & EP 2452609 A1 & CN 102548463 A | 17 |
| A | WO 2008/007771 A1 (Hitachi Metals, Ltd.), 17 January 2008 (17.01.2008), paragraphs [0073] to [0076]; fig. 19 & EP 2042094 A1 & JP 5003681 B & US 2009/0076324 A1 | 1-6,9 |

Form PCT/ISA/210 (continuation of second sheet) (July 2009)

**INTERNATIONAL SEARCH REPORT**

International application No.

PCT/JP2013/062851

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2006-68501 A  (Olympus Corp.),<br>16 March 2006 (16.03.2006),<br>fig. 1, 19 to 24, 56<br>& US 2007/0270628 A1    & EP 1773230 A<br>& WO 2006/014011 A1    & KR 10-2007-0041555 A<br>& CN 101035484 A | 12,14,15 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006068501 A
- JP 2008503310 T
- WO 2007083708 A